# EUROPEAN PATENT APPLICATION

(11) **EP 3 476 929 A1**
(43) Date of publication of application: **01.05.2019**
(21) Application number: 17820212.3
(22) Date of filing: 28.06.2017
(51) Int. Cl.: C12M 3/00, C12M 1/00, C12N 1/00

(54) **CELL ENCLOSURE DEVICE AND USE FOR SAME**

(30) Priority: 28.06.2016 JP 2016127823
(71) Applicant: National Agriculture and Food Research Organization, Tsukuba-shi, Ibaraki 305-8517 (JP)
(72) Inventor: TAKEZAWA Toshiaki, Tsukuba-shi Ibaraki 305-8602 (JP)
(74) Representative: V.O.
(86) International application number: PCT/JP2017/023754
(87) International publication number: WO 2018/003858

(57) **Abstract**

A cell enclosure device according to the present invention is a cell enclosure device for constructing a multicellular structure obtained by culturing cells, including a porous membrane in at least a portion of the cell enclosure device. In the present invention, a tissue-type chip includes the cell enclosure device in which one type of cells is enclosed. In the present invention, an organ-type chip includes the cell enclosure device in which at least two types of cells are enclosed. In the present invention, a kit for providing a multicellular structure includes an openable and closable sealed container including the tissue-type chip or the organ-type chip and a culture medium. In the present invention, an organ-type chip system includes at least two of the tissue-type chip or the organ-type chip, and the tissue-type chips or the organ-type chips are connected while maintaining a cell enclosure property. A method for culturing cells according to the present invention uses the cell enclosure device. A cell transportation method according to the present invention uses cells in the cell enclosure device.

## Description

### Technical Field

The present invention relates to a cell enclosure device, a tissue-type chip, an organ-type chip, an organ-type chip system, a method for culturing cells using the cell enclosure device, and a cell transportation method using the cell enclosure device.

Priority is claimed on Japanese Patent Application No. 2016-127823, filed on June 28, 2016, the content of which is incorporated herein by reference.

### Background Art

Enterprises involved in the discovery of drugs and alternative methods to animal experiments purchase frozen cells from a cell bank or the like, then cryopreserve the sub-cultured and proliferated cells and prepare culture models for some of the cells in order to conduct the tests necessary to carry out the development. In other words, large amounts of money and time are consumed before the tests necessary for development are conducted.

Furthermore, "tissue-type culture models" which are closer to living bodies, rather than monolayer culture cells, are required. In addition, although therapeutic techniques using cell transplantation are being rapidly developed in the field of regenerative medicine, the development of techniques for attaching a limited number of precious cells, such as stem cells, to a transplant site is becoming an urgent issue.

Examples of techniques related to "tissue-type culture models" include, for example, various gel embedding culturing techniques, spheroid culturing techniques (refer to, for example, Patent Document 1), various chamber culturing techniques (refer to, for example, Patent Document 2), and the like.

In addition, examples of techniques related to "medical cell transplantation devices" include a hydrogel membrane attached-type cell sheet transplantation technique (refer to, for example, Patent Document 3), a microencapsulation technique (refer to, for example, Patent Document 4), an atelocollagen gel embedding technique (refer to, for example, Patent Document 5), and the like.

### Citation List

### Patent Literature

[Patent Document 1] Japanese Unexamined Patent Application, First Publication No. 2012-65555
[Patent Document 2] Republished PCT International Publication No. WO2008/130025
[Patent Document 3] Japanese Unexamined Patent Application, First Publication No. 2015-35978
[Patent Document 4] Published Japanese Translation No. 2002-538194 of the PCT International Publication
[Patent Document 5] Republished PCT International Publication No. WO2006/011296

### Summary of Invention

### Technical Problem

In the related art techniques related to the "tissue-type culture models" described in Patent Documents 1 and 2, all of the culturing operations are complicated, and money and time are required to construct tissue-type culture models. Not only that, but since the production reproducibility is not always good, there are also differences between lots. Furthermore, culturing techniques in which the cells are in an exposed state, such as the spheroid culturing technique, do not always have a good protection performance with regard to the individual cells forming three-dimensional tissue.

In addition, in the related art related to the "medical cell transplantation device" described in Patent Documents 3, 4, and 5, the hydrogel membrane attached-type cell sheet transplantation technique did not always have a good cell protection performance since the cell sheet was in an exposed state. On the other hand, the microencapsulation technique and the atelocollagen gel embedding technique, which were excellent in cell protection performance, have the following problems. That is, in the microencapsulation technique, the operations are complicated and use thereof was only possible for limited cells such as pancreatic islets. In addition, in the atelocollagen gel embedding technique, the cell engraftment at the transplant site was not always good.

In addition, according to the related art techniques related to the "tissue-type culture models" and "medical cell transplantation devices" described above, it is difficult to maintain the cells in the culturing state for long periods and there is a time restriction in that the culture model or regenerated tissue has to be used immediately after the culture model or regenerated tissue for transplantation is constructed.

The present invention was made in view of the above circumstances and provides a cell enclosure device which is not only excellent in cell protection performance but which is also easy to handle and which makes the culturing of cells for long periods possible.

### Solution to Problem

As a result of intensive research to solve the above problems, the present inventors found that producing a cell enclosure device provided with a porous membrane such as collagen and enclosing and culturing cells in the cell enclosure device makes it possible to obtain high-performance tissue-type chips for which the handling is easy.

That is, the present invention includes the following aspects.

A cell enclosure device according to a first aspect of the present invention for constructing a multicellular structure obtained by culturing cells, includes a porous membrane in at least a portion of the cell enclosure device.

The porous membrane may be a semipermeable membrane having liquid-tightness in a gas phase and a semipermeable property in a liquid phase.

In the cell enclosure device of the aspect described above, multicellular cells suspended in a culture medium may be injected and an internal volume may be 10 mL or less.

In the cell enclosure device of the aspect described above, the entire device may be formed of the semipermeable membrane.

The semipermeable membrane may be formed of a material having biocompatibility.

The material having biocompatibility may be a component derived from an extracellular matrix available for gelatiion.

The component derived from an extracellular matrix available for gelation may be native collagen or atelocollagen.

A tissue-type chip according to a second aspect of the present invention includes the cell enclosure device according to the first aspect, in which one type of cells is enclosed.

A density of the cells may be 2.0 × 10³ cells/mL or more and 1.0 × 10⁹ cells/mL or less.

An organ-type chip according to a third aspect of the present invention includes the cell enclosure device according to the first aspect, in which at least two types of cells are enclosed.

The density of the cells may be 2.0 × 10³ cells/mL or more and 1.0 × 10⁹ cells/mL or less.

A kit according to a fourth aspect of the present invention for providing a multicellular structure, includes an openable and closable sealed container including the tissue-type chip according to the second aspect described above or the organ-type chip according to the third aspect described above, and a culture medium.

An organ-type chip system according to a fifth aspect of the present invention includes at least two of the tissue-type chip according to the second aspect described above, or the organ-type chip according to the third aspect described above, in which the tissue-type chips or the organ-type chips are connected while maintaining a cell enclosure property.

A method for culturing cells according to a sixth aspect of the present invention includes using the cell enclosure device according to the first aspect described above.

A cell transportation method according to a seventh aspect of the present invention includes using the cell enclosure device according to the first aspect.

### Advantageous Effects of Invention

According to the aspects described above, it is possible to provide a cell enclosure device which is not only excellent in cell protection performance but which is also easy to handle and which makes the culturing of cells for long periods possible.

### Brief Description of Drawings

FIG. 1 is a perspective view schematically showing a cell enclosure device according to a first embodiment of the present invention.
FIG. 2 is a perspective view schematically showing a cell enclosure device according to a second embodiment of the present invention.
FIG. 3 is a perspective view schematically showing a cell enclosure device according to a third embodiment of the present invention.
FIG. 4(A) is a perspective view schematically showing an organ-type chip system according to the first embodiment of the present invention. FIG. 4(B) is a perspective view schematically showing an organ-type chip system according to the second embodiment of the present invention. FIG. 4(C) is a perspective view schematically showing an organ-type chip system according to the third embodiment of the present invention. FIG. 4(D) is a perspective view schematically showing an organ-type chip system according to a fourth embodiment of the present invention.
FIG. 5 is an image showing a cell enclosure device produced in Production Example 1.
FIG. 6(A) is an image showing a state in which HepG2 cells in Example 1 are injected into the cell enclosure device produced in Production Example 1. FIG. 6(B) is an image showing the state of culturing the cell enclosure device produced in Production Example 1 in which HepG2 cells in Example 1 were enclosed.
FIG. 7(A) is an image showing the state of HepG2 cells 6 hours after the start of culturing in Example 1. FIG. 7(B) is an image showing the state of HepG2 cells on day 1 of culturing in Example 1. FIG. 7(C) is an image showing the state of HepG2 cells on day 2 of culturing in Example 1. FIG. 7(D) is an image showing the state of HepG2 cells on day 7 of culturing in Example 1. FIG. 7(E) is an image showing the state of HepG2 cells on day 12 of culturing in Example 1. FIG. 7(F) is an image showing the state of HepG2 cells on day 14 of culturing in Example 1.

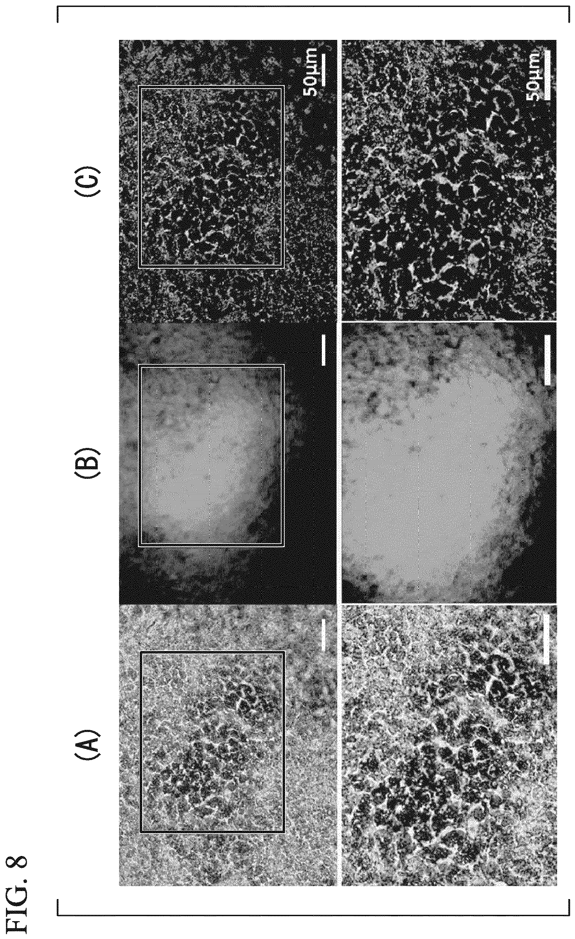

FIG. 8(A) is an image showing results observed using a phase contrast microscope after fluorescein diacetate (FD) was taken for 1 hour into a hepatic tissue-type chip of HepG2 cells on day 7 of culturing in Example 2 and then washed and further cultured for 1 hour. The lower part is an enlarged image of the portion surrounded by the square in the upper part. FIG. 8(B) is an image showing results observed using a fluorescence microscope for the pharmacokinetics of fluorescein, which is a metabolite, after FD was taken for 1 hour into the hepatic tissue-type chip of HepG2 cells on day 7 of culturing in Example 2 and then washed and further cultured for 1 hour. The lower part is an enlarged image of the portion surrounded by the square in the upper part. FIG. 8(C) is a merged image of the images (A) and (B). The lower part is an enlarged image of the portion surrounded by the square in the upper part.

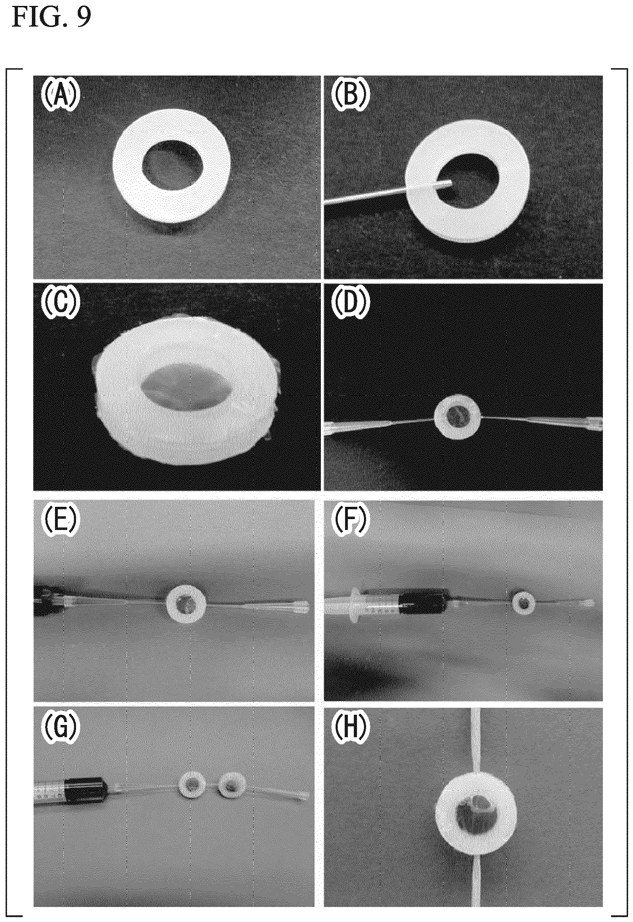

FIG. 9(A) is an image showing a 3 mm-thick silicone membrane cut out into a ring shape (inner diameter: 11 mm, outer diameter: 20 mm) in Production Example 2. FIG. 9(B) is an image showing a ring-shaped silicone membrane having holes opened in two places by passing a stainless-steel pipe through a side surface in Production Example 2. FIG. 9(C) is an image showing a cell enclosure device in which a collagen Vitrigel (registered trademark) membrane dried body is pasted on a top surface and a bottom surface of the ring-shaped silicone membrane in Production Example 2. FIG. 9(D) is an image showing a cell enclosure device in which a gel loading chip is passed through a side surface in Production Example 2. FIG. 9(E) is an image showing a state where a culture medium is injected into the inside of the cell enclosure device in Production Example 2. FIG. 9(F) is an image showing a state where a culture medium is injected into the cell enclosure device in Production Example 2. FIG. 9(G) is an image showing a state where a culture medium is passed through two cell enclosure devices connected by a tube in Production Example 2. FIG. 9(H) is an image showing a cell enclosure device plugged by a toothpick after the injection of a culture medium in Production Example 2.
FIG. 10A is an image showing a state where a suspension of HepG2 cells is injected into a cell enclosure device in Production Example 3.
FIG. 10B is an image showing a state of a cell enclosure device in which a suspension of HepG2 cells is enclosed in Production Example 3.
FIG. 11A is phase contrast microscope images and fluorescence microscope images (images stained by calcein-AM and ethidium homodimer-1) of control and hepatic tissue-type chip on day 4 of culturing in Example 3.
FIG. 11B is phase contrast microscope images and fluorescence microscope images (images stained by calcein-AM and ethidium homodimer-1) of control and hepatic tissue-type chip on day 32 of culturing in Example 3.
FIG. 12A is an image showing results observed using a phase contrast microscope after FD was taken for 1 hour into the hepatic tissue-type chip of HepG2 cells on day 32 of culturing in Example 4 and then washed and further cultured for 1 hour.
FIG. 12B is an image showing results observed using a fluorescence microscope for the pharmacokinetics of fluorescein, which is a metabolite, after FD was taken for 1 hour into the hepatic tissue-type chip of HepG2 cells on day 32 of culturing in Example 4 and then washed and further cultured for 1 hour.
FIG. 13A is a graph showing results of measuring albumin synthesis activity of control and hepatic tissue-type chips on days 4, 16, and 32 of culturing in Example 5.
FIG. 13B is a graph showing results of measuring urea synthesis activity of control and hepatic tissue-type chips on days 4, 16, and 32 of culturing in Example 5.
FIG. 13C is a graph showing results of measuring CYP3A4 activity of control and hepatic tissue-type chips on days 3, 14, and 28 of culturing in Example 5.
FIG. 14A is an image showing a state where a suspension of human dermal fibroblasts is injected into a cell enclosure device in Production Example 4.
FIG. 14B is an image showing a state of a cell enclosure device in which a suspension of human dermal fibroblasts is enclosed in Production Example 4.
FIG. 15 is phase contrast microscope images of a dermal tissue-type chip on days 1, 2, 3, and 8 of culturing in Example 6.
FIG. 16 is a phase contrast microscope image and a fluorescence microscope image (images stained by calcein-AM and ethidium homodimer-1) of the dermal tissue-type chip on day 100 of culturing in Example 6.
FIG. 17A is a phase contrast microscope image of human dermal fibroblasts on day 2 of culturing, collected and cultured from the dermal tissue-type chip on day 15 of culturing in Example 7.
FIG. 17B is a phase contrast microscope image of human dermal fibroblasts on day 7 of culturing, collected and cultured from the dermal tissue-type chip on day 15 of culturing in Example 7.
FIG. 18A is a phase contrast microscope image of human dermal fibroblasts 30 minutes after the start of culturing, collected and cultured from the dermal tissue-type chip on day 21 of culturing in Example 8.
FIG. 18B is a phase contrast microscope image of human dermal fibroblasts on day 1 of culturing, collected and cultured from the dermal tissue-type chip on day 21 of culturing in Example 8.
FIG. 19 is phase contrast microscope images and fluorescence microscope images (images stained by calcein-AM and ethidium homodimer-1) of control and hepatic tissue-type chip on day 28 of culturing in Example 9.
FIG. 20A is an image showing results observed using a phase contrast microscope after FD was taken for 1 hour into a hepatic tissue-type chip of HepG2 cells on day 35 of culturing in Example 10 and then washed and further cultured for 1 hour.
FIG. 20B is an image showing results observed using a fluorescence microscope for the pharmacokinetics of fluorescein, which is a metabolite, after FD was taken for 1 hour into the hepatic tissue-type chip of HepG2 cells on day 35 of culturing in Example 10 and then washed and further cultured for 1 hour.
FIG. 21A is a graph showing results of measuring the albumin synthesis activity of control and hepatic tissue-type chips on days 3, 7, 14, 21, and 28 of culturing in Example 11.
FIG. 21B is a graph showing results of measuring the urea synthesis activity of control and hepatic tissue-type chips on days 3, 7, 14, 21, and 28 of culturing in Example 11.
FIG. 21C is a graph showing results of measuring the CYP3A4 activity of control and hepatic tissue-type chips on days 3, 7, 14, 21, and 28 of culturing in Example 11.
FIG. 22 is a graph showing results of measuring the amount of protein permeated over time from the inside to the outside of the cell enclosure devices 4 and 5 in Example 12.
FIG. 23 is a graph showing results of measuring the amount of protein permeated from the inside to the outside of the cell enclosure devices 4, 6-1, and 6-2 in Example 13.
FIG. 24A is a fluorescence microscope image of a hepatic tissue-type chip 4 on day 1 of culturing at 37°C and on days 1, 2, 3, and 6 of storage at 25°C in Example 14. Above is an image stained by calcein-AM. In addition, below is an image stained by ethidium homodimer-1.
FIG. 24B is a fluorescence microscope image of a hepatic tissue-type chip 6-1 on day 1 of culturing at 37°C and on days 1, 2, 3, and 6 of storage at 25°C in Example 14. Above is an image stained by calcein-AM. In addition, below is an image stained by ethidium homodimer-1.
FIG. 25A is a fluorescence microscope image of a hepatic tissue-type chip 4 after post-culturing at 37°C for 1 day (24 hours) after days 1, 2, 3, and 6 of storage at 25°C in Example 15. Above is an image stained by calcein-AM. In addition, below is an image stained by ethidium homodimer-1.
FIG. 25B is a fluorescence microscope image of a hepatic tissue-type chip 6-1 after post-culturing at 37°C for 1 day (24 hours) after days 1, 2, 3, and 6 of storage at 25°C in Example 15. Above is an image stained by calcein-AM. In addition, below is an image stained by ethidium homodimer-1.
FIG. 26 is a graph showing the cellular viability of a hepatic tissue-type chip 4 and a hepatic tissue-type chip 6-1 after post-culturing at 37°C for 1 day (24 hours) after days 1, 2, 3, and 6 of storage at 25°C in Example 15.
FIG. 27A is an image showing a cell enclosure device with an indwelling needle catheter in Production Example 7.
FIG. 27B is an image showing a state where a suspension of human dermal fibroblasts is injected into a cell enclosure device in Production Example 7.
FIG. 28 is images photographing a dermal tissue-type chip on day 1 of culturing in Example 16. The left of the diagram is an image showing the dermal tissue-type chip on day 1 of culturing in the culture medium and the right of the diagram is an image of the dermal tissue-type chip on day 1 of culturing extracted from the culture medium.
FIG. 29 is phase contrast microscope images of a dermal tissue-type chip on days 0, 1, 2, and 3 of culturing in Example 16.
FIG. 30A is an image showing a cell enclosure device formed only of an atelocollagen Vitrigel (registered trademark) membrane dried body in Production Example 8.
FIG. 30B is an image showing a state where a suspension of human dermal fibroblasts is injected into a cell enclosure device in Production Example 8.
FIG. 31 is an image photographing a dermal tissue-type chip on day 1 of culturing in Example 17. The left of the diagram is an image showing the dermal tissue-type chip on day 1 of culturing in the culture medium and the right of the diagram is an image of the dermal tissue-type chip on day 1 of culturing extracted from the culture medium.
FIG. 32 is phase contrast microscope images of a dermal tissue-type chip on days 0, 1, 2, and 3 of culturing in Example 17.

### Description of Embodiments

A more detailed description will be given below of the present invention with reference to embodiments; however, the present invention is not at all limited to the following embodiments.

### <<Cell Enclosure Device>>

The cell enclosure device of the present embodiment is for constructing a multicellular structure obtained by culturing cells and includes a porous membrane in at least a portion thereof.

It is possible to easily handle the cell enclosure device of the present embodiment with tweezers or the like. In addition, in the related art, there is a time restriction in that, after a multicellular structure is produced as a culture model or regenerated tissue for transplantation, the multicellular structure has to be used within 1 to 3 days. In contrast, the cell enclosure device of the present embodiment is able to culture cells for a long period of approximately 3 to 30 days, and is not subject to a time restriction.

In the present specification, the term "porous membrane" means a membrane having many pores, and encompasses membranes having voids and membranes having pores and voids.

In the cell enclosure device of the present embodiment, for example, in a case where the cell enclosure device of the present embodiment in which cells are enclosed is placed in a container including a culture medium, cells are not able to permeate to the outside of the cell enclosure device. On the other hand, nutrients dissolved in the culture medium are able to permeate to the inside of the cell enclosure device and cell products including waste matter dissolved in the culture medium are able to permeate to the outside of the cell enclosure device. Therefore, it is possible to use the cell enclosure device of the present embodiment for the culturing of cells for long periods.

In the present specification, "multicellular structure" means a three-dimensional structure formed of monolayer cells or multi-layered cells in which a plurality of cells form cell-substratum bonds and cell-cell bonds. The multicellular structure in the present embodiment is formed of one or more types of functional cells and a substratum which has the role of a scaffold. That is, in the multicellular structure in the present embodiment, a plurality of functional cells interacts with a substratum to construct a form which is more similar to tissues or organs in a living body. Accordingly, capillary network-like structures such as blood vessels and/or bile ducts may be three-dimensionally constructed in the multicellular structure. Such capillary network-like structures may be formed only inside the multicellular structure, or may be formed such that at least a portion thereof is exposed on the surface or the bottom surface of the multicellular structure.

### <Structure>

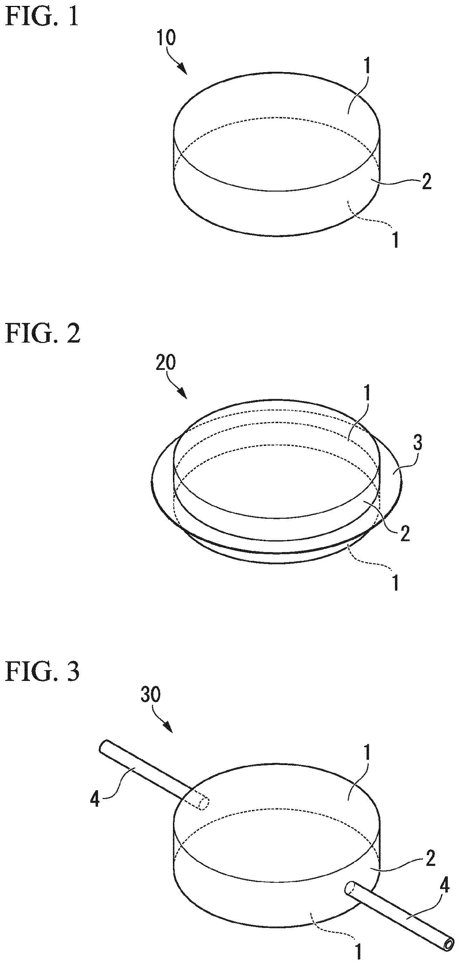

FIG. 1 is a perspective view schematically showing a cell enclosure device according to a first embodiment of the present invention.

The cell enclosure device 10 shown here is provided with a porous membrane 1 on a top surface and a bottom surface, and has a cylindrical shape sealed on the side surface by a member 2. In FIG. 1, an exemplary example of the porous membrane is provided on the top surface and the bottom surface, but the porous membrane may be provided on a part of the top surface, the bottom surface, the side surface, or the like. Alternatively, the entirety of the top surface, the bottom surface, the side surface, and the like may be formed of a semipermeable membrane described below, among the porous membranes. Among these, in a case where the cell enclosure device of the present embodiment is used as a culture model, a porous membrane is preferably provided on the top surface and the bottom surface. In addition, in a case of being used as a medical cell transplantation device, the entirety thereof is preferably formed of a semipermeable membrane.

In FIG. 1, the cell enclosure device is shown to have a cylindrical shape, but the cell enclosure device may have other shapes. The shape of the cell enclosure device of the present embodiment may be any shape as long as it is possible for cells to be enclosed and oxygen and nutrients uniformly dissolved in the culture medium are distributed to the cells. In addition, in the cell enclosure device of the present embodiment, the inside of the device may be filled with the culture medium, or a gas portion may be left without being filled with the culture medium. Examples of the shape of the cell enclosure device of the present embodiment include a cylindrical shape, a circular cone, a circular truncated cone, a pyramid, a truncated pyramid, a sphere, a polyhedron (for example, a tetrahedron, a pentahedron, a hexahedron (including cubes), an octahedron, a dodecahedron, an icosahedron, an icositetrahedron, a Kepler-Poinsot polyhedron, or the like), and the like, without being limited thereto.

In a case where the shape of the cell enclosure device is a cylinder as shown in FIG. 1, the inner diameter of the cell enclosure device is preferably 1 mm or more and 60 mm or less, more preferably 3 mm or more and 35 mm or less, and even more preferably 5 mm or more and 26 mm or less.

In addition, the outer diameter of the cell enclosure device is preferably 3 mm or more and 68 mm or less, more preferably 5 mm or more and 43 mm or less, and even more preferably 7 mm or more and 32 mm or less.

In addition, the thickness of the cell enclosure device (cylinder height) is 5 µm or more, preferably 50 µm or more and 15 mm or less, more preferably 100 µm or more and 10 mm or less, and even more preferably 200 µm or more and 2.5 mm or less.

In the present specification, the "thickness of the cell enclosure device (cylinder height)" means the distance from the outer edge of the top surface of the cell enclosure device to the outer edge of the bottom surface.

Although the top surface and the bottom surface are shown to be flat in FIG. 1, the top surface and the bottom surface may have a concave structure or a convex structure. In particular, when the top surface and the bottom surface have a concave structure, the center portion of the concave portion on the inner side of the top surface (the most concave portion on the inner side of the top surface) and the center portion of the concave portion on the inner side of the bottom surface (the most concave portion on the inner side of the bottom surface) preferably do not come into contact and are maintained at a certain distance (for example, 5 µm or more). Due to this, the thickness of the cell enclosure device (cylinder height), that is, the distance from the outer edge of the top surface of the cell enclosure device to the outer edge of the bottom surface is longer than the distance from the center portion of the concave portion on the outside of the top surface (the most concave portion on the outside of the top surface) to the center portion of the concave portion on the outside of the bottom surface (the most concave portion on the outside of the bottom surface). Due to this, for example, in a case where a medicine is added from the top surface, it is possible to maintain the directionality of the added medicine. Furthermore, since the top surface and the bottom surface have a concave structure, it is possible to newly seed and culture cells outside the top surface and the bottom surface.

The internal volume of the cell enclosure device of the present embodiment may be small scale as long as it is possible to inject multicellular cells suspended in a culture medium and to construct a multicellular structure to be used in an in vitro test system such as a test for assaying cell activity. Specifically, for example, the internal volume is preferably 10 mL or less, more preferably 10 µL or more and 5 mL or less, even more preferably 15 µL or more and 2 mL or less, and particularly preferably 20 µL or more and 1 mL or less. The internal volume being the upper limit value or less means that oxygen and culture medium nutrients are sufficiently supplied, and makes it possible to efficiently culture the cells over a long period. In addition, the internal volume being the lower limit value or more makes it possible to obtain cells having a sufficient number of cells and cell density for use in an in vitro test system.

FIG. 2 is a perspective view schematically showing a cell enclosure device according to a second embodiment of the present invention.

In the diagrams following FIG. 2, the same reference numerals are given to the same constituent elements as in the cases of the already explained diagrams and detailed descriptions thereof will be omitted.

A cell enclosure device 20 shown here is the same as the cell enclosure device 10 shown in FIG. 1 except for being provided with a support 3. That is, the cell enclosure device 20 is provided with the porous membrane 1 on the top surface and the bottom surface, has the shape of a cylinder sealed on the side surface by the member 2, and is provided with the support 3 on the outside surface.

Having the support 3 makes it possible for the cell enclosure device 20 to culture the cells in a gas phase by, for example, fixing the cell enclosure device 20 in which the cells are enclosed in a larger container. In addition, the cell enclosure device 20 having the support 3 means that, for example, the cell enclosure device 20 in which cells are enclosed is able to float due to the buoyancy in the culture medium. In addition, in a case where the porous membrane 1 is provided on the top surface and the bottom surface as in the cell enclosure device 20, since the top surface is in contact with air and the bottom surface is in contact with the culture medium, it is possible to culture the cells in the gas phase and the liquid phase. In addition, the support 3 may be fixed to the cell enclosure device 20 or may be detachable.

FIG. 3 is a perspective view schematically showing a cell enclosure device according to a third embodiment of the present invention.

The cell enclosure device 30 shown here is the same as the cell enclosure device 10 shown in FIG. 1 except for being provided with tubes 4. That is, the cell enclosure device 30 is provided with the porous membrane 1 on the top surface and the bottom surface, and has the shape of a cylinder sealed on the side surface by the member 2. Furthermore, the tubes 4 are provided so as to face the outer surface of the cell enclosure device 30, the tubes 4 are inserted inside the cell enclosure device 30, and two of the tubes 4 and the cell enclosure device 30 are in communication.

Providing the tube 4 makes it possible for the cell enclosure device 30 to also supply the culture medium from the side surface. Furthermore, connecting the cell enclosure devices 30 provided with the tubes 4 to each other makes it possible to construct the organ-type chip system described below.

In addition, an openable and closable device (not shown) such as a plug or a valve is preferably provided at the end of the opposite side to the side where the tube 4 is inserted into the cell enclosure device 30.

The cell enclosure device of the present embodiment is not limited to those devices shown in FIG. 1 to FIG. 3, but parts of the configurations shown in FIG. 1 to FIG. 3 may be changed or removed or other configurations may be added to the embodiments previously described, within a range in which the effect of the cell enclosure device of the present embodiment is not impaired.

For example, in the cell culture device shown in FIG. 1 and FIG. 2, the member may be provided with an injection hole. In a case where the injection hole is provided, a plug for closing the injection hole is preferably provided.

The shape of the injection hole is not particularly limited, and examples thereof include a circular shape, a polygonal shape (including regular polygonal shapes and the like), an elliptical shape, and the like.

The radius of the injection hole may be appropriately adjusted according to the thickness of the cell culture device (that is, the member height), and may be, for example, 10 µm or more and 1000 µm or less.

In addition, in the cell enclosure device of the present embodiment, it is possible to arbitrarily adjust the size and shape of each configuration (porous membrane, member, or the like) according to the purpose.

### <Configurations>

### [Porous Membrane]

The porous membrane used in the cell enclosure device of the present embodiment is not particularly limited as long as the membrane has holes of a size such that cells enclosed therein do not permeate to the outside. Examples of the porous membrane include, filter paper, semipermeable membranes (for example, ultrafiltration membranes or the like), non-woven fabric, gauze-like mesh, various membrane filters, and the like, without being limited thereto.

In addition, the pore size of the porous membrane in the present embodiment may be, for example, 0.01 µm or more and 1,500 µm or less, for example, 0.01 µm or more and 1.0 µm or less, or, for example, 0.01 µm or more and 0.45 µm or less. The hole size may be appropriately selected according to the size of the cells to be enclosed therein or of small living organisms to be described below.

In particular, the porous membrane in the present embodiment is preferably a semipermeable membrane having liquid-tightness in a gas phase and having a semipermeable property in a liquid phase. Since the semipermeable membrane has liquid-tightness in the gas phase, for example, in a case where a liquid such as a culture medium is included in the cell enclosure device of the present embodiment, the liquid does not leak in the gas phase and it is possible to maintain the liquid inside. This liquid-tightness is due to the surface tension of the semipermeable membrane. On the other hand, since it is possible for a gas to pass through, in a case where a liquid is included inside, the liquid inside evaporates over time.

In addition, since the semipermeable membrane used in the cell enclosure device of the present embodiment has a semipermeable property in the liquid phase, for example, in a case where the cell enclosure device of the present embodiment in which the cells are enclosed is placed in a container including a culture medium, the cells in the cell enclosure device do not permeate to the outside of the device, while the nutrients dissolved in the culture medium are able to permeate to the inside of the cell enclosure device and cell products including waste matter dissolved in the culture medium are able to permeate to the outside of the cell enclosure device. Therefore, it is possible to use the cell enclosure device of the present embodiment for the culturing of cells for long periods.

More specifically, it is sufficient if the semipermeable membrane used in the cell enclosure device of the present embodiment, for example, allows a polymer compound having a molecular weight of approximately 1,000,000 or less to permeate therethrough, or, for example, allows a molecular compound having a molecular weight of approximately 200,000 or less to permeate therethrough.

In the present specification, "liquid-tightness" means a state in which liquid does not leak. In addition, in the present specification, "a semipermeable property" means a property capable of allowing only molecules or ions having a certain molecular weight or less to permeate therethrough, and a "semipermeable membrane" is a membrane having such a property.

The material of the porous membrane may be any material which is not cytotoxic, and may be a natural polymer compound or may be a synthetic polymer compound. In addition, in a case where the porous membrane is a semipermeable membrane, the material thereof is preferably a material having biocompatibility, and more preferably a material having bioabsorbability. In a case where the cell enclosure device of the present embodiment is entirely formed of a semipermeable membrane having biocompatibility or bioabsorbability, utilization is possible as a medical cell transplantation device.

In the present specification, "biocompatibility" means an evaluation criterion indicating compatibility between living tissue and a material. In addition, "having biocompatibility" means a state in which the material itself has no toxicity, does not have components derived from microorganisms such as endotoxins, does not physically stimulate the living tissue and is not rejected even when interacting with a protein, a cell, or the like which forms living tissue. In addition, in the present specification, "bioabsorbability" means a property that a material retains a shape or physical properties for a certain period in a living body and then disappears from the place of introduction into the living body by being decomposed and absorbed.

Examples of natural polymer compound include components derived from an extracellular matrix available for gelatiion, polysaccharides (for example, alginate, cellulose, dextran, pullulane, polyhyaluronic acid, derivatives thereof, and the like), chitin, poly(3-hydroxyalkanoate) (in particular, poly(β-hydroxybutyrate), poly(3-hydroxyoctanoate)), poly(3-hydroxy fatty acid), fibrin, agar, agarose, and the like, without being limited thereto.

The cellulose also includes cellulose modified by synthesis, and examples thereof include cellulose derivatives (for example, alkyl cellulose, hydroxyalkyl cellulose, cellulose ether, cellulose ester, nitrocellulose, chitosan, or the like) and the like. More specific examples of cellulose derivatives include methylcellulose, ethylcellulose, hydroxypropylcellulose, hydroxypropylmethylcellulose, hydroxybutylmethylcellulose, cellulose acetate, cellulose propionate, cellulose acetate butyrate, cellulose acetate phthalate, carboxymethylcellulose, cellulose triacetate, cellulose sulfate sodium salt, and the like.

Among these, the natural polymer compound is preferably a component derived from an extracellular matrix available for gelation, fibrin, agar, or agarose since these have excellent water retention.

Examples of components derived from an extracellular matrix available for gelation include collagen (type I, type II, type III, type V, type XI, or the like), a reconstituted basement membrane component (trade name: Matrigel) derived from mouse EHS tumor extract (including type IV collagen, laminin, heparan sulfate proteoglycan, or the like), glycosaminoglycan, hyaluronic acid, proteoglycans, gelatin, and the like, without being limited thereto. It is possible to produce porous membranes (in particular, semipermeable membranes) by selecting components suitable for gelation such as salts, the concentrations and pH thereof, and the like. In addition, combining raw materials makes it possible to obtain a porous membrane (in particular, a semipermeable membrane) imitating various tissues in living bodies.

Examples of synthetic polymer compounds include polyphosphazene, poly(vinyl alcohol), polyamide (such as nylon), polyester amide, poly(amino acid), polyanhydride, polysulfone, polycarbonate, polyacrylate (acrylic resin), polyalkylene (for example, polyethylene and the like), polyacrylamide, polyalkylene glycol (for example, polyethylene glycol and the like), polyalkylene oxide (for example, polyethylene oxide and the like), polyalkylene terephthalate (for example, polyethylene terephthalate and the like), polyorthoester, polyvinyl ether, polyvinyl ester, polyvinyl halide, polyvinyl pyrrolidone, polyester, polysiloxane, polyurethane, polyhydroxy acid (for example, polylactide, polyglycolide, and the like), poly(hydroxybutyric acid), poly(hydroxyvaleric acid), poly[lactide-co-(ε-caprolactone)], poly[glycolide-co(ε-caprolactone)], poly(hydroxyalkanoate), copolymers thereof, and the like, without being limited thereto.

More specific examples of the polyacrylate (acrylic resin) include poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), and the like.

Among these, as the synthetic polymer compound, polyhydroxy acids (for example, polylactide, polyglycolide, and the like), polyethylene terephthalate, poly(hydroxybutyric acid), poly(hydroxyvaleric acid), poly[lactide-co-(ε-caprolactone)], poly[glycolide-co(ε-caprolactone)], poly(hydroxyalkanoate), polyorthoester, or copolymers thereof are preferable since these have bioabsorbability.

The material of the porous membrane in the present embodiment may be formed of one type of the exemplary examples of materials above, or may be formed of two or more types thereof. In addition, the material of the porous membrane in the present embodiment may be formed of any of natural polymer compounds or synthetic polymer compounds, or may be formed of both natural polymer compounds and synthetic polymer compounds.

Among these, in a case where the porous membrane in the present embodiment is a semipermeable membrane, a natural polymer compound is preferable as the material thereof, a component derived from an extracellular matrix available for gelation is more preferable, and collagen is even more preferable. In addition, examples of more preferable materials among collagens include native collagen or atelocollagen.

In a case where the material of the porous membrane in the present embodiment is a component derived from an extracellular matrix, the component derived from an extracellular matrix is preferably contained in an amount of 0.1 mg or more and 10.0 mg or less per 1 cm² unit area of the porous membrane (in particular, the semipermeable membrane), and more preferably contained in an amount of 0.5 mg or more and 5.0 mg or less. In particular, in a case where the component derived from the extracellular matrix is atelocollagen, atelocollagen is preferably contained in an amount of 0.5 mg or more and 10.0 mg or less per 1 cm² of the unit area of the porous membrane (in particular, the semipermeable membrane), and more preferably contained in an amount of 2.5 mg or more and 5.0 mg or less per 1 cm².

The amount of the component derived from the extracellular matrix (in particular, atelocollagen) in the porous membrane (in particular, the semipermeable membrane) being in the ranges described above makes it possible to have strength such that it is possible to inject and culture the cells in the cell enclosure device. The "weight per unit area of 1 cm² of the membrane" refers to the weight of the component contained per 1 cm² of the material piece, with the thickness of the membrane being arbitrary.

The thickness of the porous membrane in the present embodiment is not particularly limited; however, the thickness is preferably 1 µm or more and 1000 µm or less, more preferably 1 µm or more and 500 µm or less, even more preferably 5 µm or more and 300 µm or less, and particularly preferably 10 µm or more and 200 µm or less. The thickness of the porous membrane being within the above range makes it possible to have strength such that it is possible to inject and culture the cells in the cell enclosure device. In addition, in a case where the porous membrane is a semipermeable membrane, the thickness of the porous membrane being within the above range makes it possible to suitably use the cell enclosure device of the present embodiment as a medical cell transplantation device.

In addition, there is no concern that the porous membrane of the present embodiment will break during use and the porous membrane is excellent in practical use. In particular, in a case of being used for a medical cell transplantation device, the strength of the porous membrane (in particular, the semipermeable membrane) is enough to withstand the transplant operation.

### (Production Method of Porous Membrane)

### 1. Method for Producing a Porous Membrane using Synthetic Polymer Compound

For example, in a case where the constituent material of the porous membrane is the synthetic polymer compound described above, it is possible to produce the porous membrane using a known method (for example, refer to Japanese Unexamined Patent Application, First Publication No. 2001-149763 or the like).

More specifically, as a method for producing a porous membrane using a synthetic polymer compound, first, a membrane-forming stock solution in which a synthetic polymer compound is dissolved in an organic solvent is prepared.

As the organic solvent, any solvent suitable for the synthetic polymer compound may be used, and examples thereof include tetrahydrofuran, dioxane, dimethylformamide, dimethylacetamide, N-methyl-2-pyrrolidone, and the like, without being limited thereto.

The mixing ratio of the synthetic polymer compound and the organic solvent may be appropriately adjusted according to the types of the synthetic polymer compound and the organic solvent to be used, for example, the synthetic polymer compound may be 15% by weight and the organic solvent may be 85% by weight. In addition, the temperature of the organic solvent at the time of dissolution may be usually 30°C or more and 100°C or less, and preferably 50°C or more and 80°C or less.

Next, using, for example, a method of discharging from a nozzle, the prepared membrane-forming stock solution is coagulated in a coagulating liquid and a porous membrane with a predetermined shape is produced.

As the coagulating liquid, a mixed solution of an organic solvent and water is preferably used. As the organic solvent used for the coagulating liquid, it is possible to use the same organic solvents which are exemplary examples of the organic solvent used for dissolving the synthetic polymer compound. The organic solvent used for the coagulating liquid may be of the same type as the organic solvent used for dissolving the synthetic polymer compound or may be of a different type.

In addition, the ratio of water in the coagulating liquid may be, for example, 30% by weight or more and 80% by weight or less.

Furthermore, for the purpose of adjusting the coagulation rate, alcohols such as methanol, ethanol, isopropanol, and glycerin, and glycols such as ethylene glycol and propylene glycol may be added to the coagulating liquid.

The obtained porous membrane may be used after washing with distilled water or the like and performing further sterilization by ultraviolet irradiation or the like.

### 2. Method for Producing a Porous Membrane using Hydrogel

In addition, for example, in a case where the constituent material of the porous membrane is a hydrogel, it is possible to produce the porous membrane using a known method (for example, refer to PCT International Publication No. WO 2012/026531, Japanese Unexamined Patent Application, First Publication No. 2012-115262, and Japanese Unexamined Patent Application, First Publication No. 2015-35978).

In the present specification, the term "hydrogel" refers to a substance in which the polymer compound has a network structure due to chemical bonding and which has a large amount of water in the network thereof. More specifically, the hydrogel means a substance obtained by introducing cross-linking into an artificial material of a natural polymer compound or synthetic polymer compound to cause gelation. Examples of hydrogels include natural polymer compounds such as the above-described component derived from the extracellular matrix available for gelation, fibrin, agar, agarose, and cellulose, and synthetic polymer compounds such as polyacrylamide, polyvinyl alcohol, polyethylene oxide, and poly(II-hydroxyethylmethacrylate)/polycaprolactone, and the like.

More specifically, as a method for producing a porous membrane using a hydrogel, first, a hydrogel which is in a state of being not completely gelled (may be referred to below as "sol") is arranged in a mold and gelation is induced.

In a case where the sol is a collagen sol, as a collagen sol having an optimal salt concentration, a collagen sol may be used which is prepared using physiological saline, phosphate buffered saline (PBS), Hank's Balanced Salt Solution (HBSS), a basic culture medium, a serum-free culture medium, a serum-containing culture medium, or the like. In addition, the pH of the solution at the time of collagen gelation may be, for example, 6 or more and 8 or less.

In particular, in a case where a serum-free culture medium is used, since it is possible to avoid including substances (for example, antigens, pathogenic factors, or the like), which are not suitable for transplantation and which are included in serum components of other animals, in the porous membrane, it is possible to obtain a porous membrane (in particular, a semipermeable membrane) suitable for a case of being used in a medical cell transplantation device.

In addition, the collagen sol may be prepared at approximately 4°C, for example. Thereafter, the preserved temperature during gelation may be lower than the denaturation temperature of collagen depending on the animal species of collagen to be used, and, generally, it is possible to perform gelation in several minutes to several hours by incubating at a temperature of 20°C or more and 37°C or less.

In addition, the concentration of the collagen sol for producing the porous membrane is preferably 0.1% or more and 1.0% or less, and more preferably 0.2% or more and 0.6% or less. When the concentration of the collagen sol is the above lower limit value or more, the gelation is not too weak, and when the concentration of the collagen sol is the above upper limit value or less, it is possible to obtain a porous membrane (in particular, a semipermeable membrane) formed of uniform collagen gel.

Furthermore, the obtained hydrogel may be dried to obtain a hydrogel dried body. Drying the hydrogel makes it possible to completely remove the free water in the hydrogel and to further proceed with partial removal of bonding water.

Furthermore, Vitrigel (registered trademark) may be obtained by rehydrating the obtained hydrogel dried body with PBS, the culture medium to be used, or the like.

As the period of this vitrification step (the step of completely removing the free water in the hydrogel and then proceeding to partially remove the bonding water) is lengthened, it is possible to obtain Vitrigel (registered trademark) with superior transparency and strength at the time of rehydration. After a short period of vitrification, it is also possible to wash the Vitrigel (registered trademark) obtained by rehydration with PBS or the like and carry out the vitrification again as necessary.

As a drying method, for example, it is possible to use various methods such as air drying, drying in a sealed container (circulating air in a container, or constantly supplying dry air), drying in an environment in which silica gel is placed, and the like. For example, examples of methods of air drying include methods such as drying for 2 days in an incubator kept sterile at 10°C and 40% humidity, or drying in a clean bench in a sterile state for one day at room temperature.

In the present specification, "Vitrigel (registered trademark)" refers to a gel in a stable state obtained by vitrification and subsequent rehydration of a hydrogel in the related art and it was the present inventors who named "Vitrigel (registered trademark)".

In addition, in the present specification, when describing in detail the steps of producing a porous membrane formed of a hydrogel, the hydrogel dried body immediately after the vitrification step and not subjected to a rehydration step is simply referred to as a "hydrogel dried body". Then, the gel obtained through the rehydration step after the vitrification step is expressed distinctly as "Vitrigel (registered trademark)". In addition, the dried body obtained by vitrifying Vitrigel (registered trademark) is referred to as "Vitrigel (registered trademark) dried body". In addition, a product obtained by subjecting a Vitrigel (registered trademark) dried body to a step of ultraviolet irradiation is referred to as a "Vitrigel (registered trademark) dried body subjected to an ultraviolet irradiation treatment". In addition, a gel obtained by carrying out a step of rehydrating the "Vitrigel (registered trademark) dried body subjected to an ultraviolet irradiation treatment" is referred to as a "Vitrigel (registered trademark) material". In addition, the dried body obtained by vitrifying the Vitrigel (registered trademark) material is referred to as a "dried body of Vitrigel (registered trademark) material". Accordingly, "Vitrigel (registered trademark)" and "Vitrigel (registered trademark) material" are hydrates.

That is, the obtained Vitrigel (registered trademark) may be re-dried to carry out re-vitrification to obtain a Vitrigel (registered trademark) dried body.

Examples of the drying method include the same methods as described above.

In addition, the obtained Vitrigel (registered trademark) dried body may be irradiated with ultraviolet rays to obtain the "Vitrigel (registered trademark) dried body subjected to an ultraviolet irradiation treatment".

For ultraviolet irradiation, it is possible to use a known ultraviolet irradiation apparatus.

The total irradiation amount per unit area of ultraviolet irradiation energy to Vitrigel (registered trademark) dried body is preferably 0.1 mJ/cm² or more and 6000 mJ/cm² or less, more preferably 10 mJ/cm² or more and 4000 mJ/cm² or less, and even more preferably 100 mJ/cm² or more and 3000 mJ/cm² or less. When the total irradiation amount is in the above range, it is possible for the transparency and strength of Vitrigel (registered trademark) material obtained in the subsequent rehydration step to be particularly preferable.

In addition, the irradiation of the Vitrigel (registered trademark) dried body with ultraviolet rays may be repeated a plurality of times. In a case of repeating the irradiation of the Vitrigel (registered trademark) dried body with ultraviolet rays, it is preferable that, after the irradiation of the first ultraviolet rays, the steps of rehydration and re-vitrification of the Vitrigel (registered trademark) dried body subjected to ultraviolet irradiation treatment are performed and then the dried body of Vitrigel (registered trademark) material after re-vitrification second and subsequent times is irradiated with ultraviolet rays.

When the total ultraviolet irradiation amount per unit area is the same, the Vitrigel (registered trademark) dried body is repeatedly irradiated with ultraviolet rays while being divided a plurality of times, such that it is possible to further increase the transparency and strength of the obtained Vitrigel (registered trademark) material in the following rehydration step. In addition, the larger the number of divisions, the better. For example, when the total irradiation amount per unit area of the ultraviolet irradiation on the Vitrigel (registered trademark) dried body is in the range of 1000 mJ/cm² or more and 4000 mJ/cm² or less, the number of times of irradiation in the above range is preferably 2 times or more and 10 times or less, and more preferably 2 times or more and 6 times or less.

In addition, in a case of repeating the irradiation of the Vitrigel (registered trademark) dried body with ultraviolet rays, the irradiation is carried out after dividing irradiation site of the ultraviolet rays into one side of the Vitrigel (registered trademark) dried body and the other side (the upper side and the lower side), and the total irradiation amount may be the total ultraviolet irradiation amount per unit area on the Vitrigel (registered trademark) dried body.

It is considered that the increase in the strength and transparency of the obtained Vitrigel (registered trademark) material in the subsequent rehydration step by irradiating the Vitrigel (registered trademark) dried body with ultraviolet rays is because the polymer compounds in the Vitrigel (registered trademark) material are cross-linked by the ultraviolet rays. In other words, it is considered that, through this operation, it is possible to maintain high transparency and strength in the Vitrigel (registered trademark) material.

Furthermore, the Vitrigel (registered trademark) material may be obtained by subjecting the obtained Vitrigel (registered trademark) dried body subjected to ultraviolet irradiation treatment to rehydration with PBS, the culture medium to be used, or the like.

Furthermore, a dried body of Vitrigel (registered trademark) material may be obtained by drying the obtained Vitrigel (registered trademark) material to carry out re-vitrification.

Examples of the drying method include the same methods as described above.

### [Member]

In the cell enclosure device of the present embodiment, members forming portions other than the porous membrane may be any members having liquid-tightness. In addition, in the cell enclosure device of the present embodiment, the member forming a portion other than the porous membrane may have air permeability or may not have air permeability.

In a case where the member has air permeability, the oxygen permeability coefficient may be, for example, 100 cm³/m² per 24 hr at 1 atm or more and 5000 cm³/m² per 24 hr at 1 atm or less, for example, 1000 cm³/m² per 24 hr at 1 atm or more and 3000 cm³/m² per 24 hr at 1 atm or less, and, for example, 1200 cm³/m² per 24 hr at 1 atm or more and 2500 cm³/m² per 24 hr at 1 atm or less. Furthermore, the carbon dioxide permeability coefficient may be, for example, 1000 cm³/m² per 24 hr at 1 atm or more and 20,000 cm³/m² per 24 hr at 1 atm or less, for example, 3000 cm³/m² per 24 hr at 1 atm or more and 15,000 cm³/m² per 24 hr at 1 atm or less, and, for example, 5000 cm³/m² per 24 hr at 1 atm or more and 10,000 cm³/m² per 24 hr at 1 atm or less. In addition, in a case where the member does not have air permeability, the oxygen permeability coefficient may be, for example, 100 cm³/m² per 24 hr at 1 atm or less, and, for example, 50 cm³/m² per 24 hr at 1 atm or less. Furthermore, the carbon dioxide permeability coefficient may be, for example, 1000 cm³/m² per 24 hr at 1 atm or less, and, for example, 500 cm³/m² per 24 hr at 1 atm or less.

In the cell enclosure device of the present embodiment, the material of members forming portions other than the porous membrane may be any material suitable for cell culturing. Examples of materials forming portions other than the porous membrane include glass materials such as soda lime glass, Pyrex (registered trademark) glass, Vycor (registered trademark) glass, and quartz glass; elastomer materials such as urethane rubber, nitrile rubber, silicone rubber, silicone resins (for example, polydimethylsiloxane), fluororubber, acrylic rubber, isoprene rubber, ethylene propylene rubber, chlorosulfonated polyethylene rubber, epichlorohydrin rubber, chloroprene rubber, styrene butadiene rubber, butadiene rubber, and polyisobutylene rubber; plastics including dendritic polymers such as poly(vinyl chloride), poly(vinyl alcohol), poly(methyl methacrylate), poly(vinyl acetate-co-maleic anhydride), poly(dimethylsiloxane) monomethacrylate, cyclic olefin polymers, fluorocarbon polymer, polystyrene, polypropylene, and polyethylenimine; copolymers of poly(vinyl acetate-co-maleic anhydride), poly(styrene-co-maleic anhydride), poly(ethylene-co-acrylic acid), and derivatives thereof, without being limited thereto.

In addition, it is possible to appropriately select the shape of the members according to the entire shape of the cell enclosure device of the present embodiment and the portion forming the cell enclosure device of the present embodiment.

In addition, the members may be changed (for example, coloring, printing, or the like) in order to identify individual cell enclosure devices.

### (Method of Producing Member)

It is possible to produce the member in the present embodiment using a known method depending on the material to be used.

In a case where an elastomer material or plastic is used as the material of the member, examples of the method of producing the member include a compression molding method, an injection molding method, an extrusion molding method, and the like, without being limited thereto.

In addition, in a case where a glass material is used as the material of the member, examples of the production method include a droplet molding method, the Danner method, an overflow method, a float method, a blow molding method, a press molding method, and the like, without being limited thereto.

### [Support]

Examples of the material of the support used in the cell enclosure device of the present embodiment include organic materials such as polyamide (for example, nylon and the like), polyolefin resin, polyester resin, polystyrene resin, polycarbonate, polyamide resin, and silicone resin; inorganic materials such as ceramics and glass, and the like, without being particularly limited.

In addition, examples of the shape of the support include a sheet shape, a rod shape, and the like, without being limited thereto.

In addition, the support may be changed (for example, coloring, printing, or the like) in order to identify individual cell enclosure devices.

### (Method of Producing Support)

It is possible to produce the support in the present embodiment by a known method depending on the material to be used.

For example, in a case where an organic material is used as the material of the support, examples of the production method include a compression molding method, a calendar molding method, an injection molding method, an extrusion molding method, inflation molding, and the like, without being limited thereto.

In addition, in a case where glass is used as the material of the support, examples of production methods include the same methods provided as exemplary examples above (method of producing a member).

In addition, in a case of using ceramics as the material of the support, examples of production methods include dry molding methods (for example, a mold forming method, a cold isostatic pressing method, a hot pressing method, a hot isostatic pressing method, or the like), plastic molding methods (for example, a wax molding method, an extrusion molding method, an injection molding method), cast molding methods (for example, a slurry casting method, a pressure casting method, a rotary casting method, or the like), a tape molding method, or the like, without being limited thereto.

### [Tube]

The material of the tube used for the cell enclosure device of the present embodiment is not particularly limited, and, for example, in a case where the cell enclosure device is utilized as a medical cell implantation device, the material of tube is preferably a material having biocompatibility. Examples of materials having biocompatibility include the natural polymer compounds and synthetic polymer compounds provided as exemplary examples of the "porous membrane" described above.

In addition, in a case where the cell enclosure device is used for constructing a multicellular structure used in an in vitro test system such as a test for assaying the activity of cells, the material may be the material having biocompatibility described above, or may be a material suitable for culturing cells. Examples of materials having biocompatibility include the natural polymer compounds and synthetic polymer compounds provided as exemplary examples of the "porous membrane" described above. Examples of the materials suitable for culturing cells include the same materials provided as exemplary examples of the [Member] described above.

In addition, more specific examples of materials suitably used as a tube include a medical catheter, an indwelling needle, and the like.

### (Method of Producing Tube)

It is possible to produce the tube in the present embodiment by a known method depending on the material to be used.

As a specific production method, a tubular shape may be formed using the same method as described in the above "Method of Producing Porous Membrane" and "Method of Producing Member" described above.

### <<Method of Producing Cell Enclosure Device>>

It is possible to produce the cell enclosure device of the present embodiment by assembling only a porous membrane, or a porous membrane and a member, so as to have a desired shape. In addition, as necessary, a support and a tube may be provided.

The production methods of each of the porous membrane, the member, the support, and the tube are as described above.

More specifically, a detailed description will be given below of the method of producing the cell enclosure device of the present embodiment shown in FIG. 1.

First, two porous membranes 1 having the same size as the top surface and the bottom surface of the member 2 or having a size one size larger than the top surface and the bottom surface of the member 2 are prepared. Next, the prepared porous membranes 1 are joined so as to become the top surface and the bottom surface of the member 2, respectively.

Examples of methods for joining the porous membrane 1 and the member 2 include a joining method using an adhesive, a joining method with a double-sided tape, a joining method by heat welding using a heat sealer, a hot plate, ultrasonic waves, a laser, or the like, a method using tenon and mortise joining by producing a tenon and a mortise (for example, single-sided, double-sided, three-sided, four-sided, small rooted, marginal, two-tenon, two-step tenon, or the like), and the like, without being limited thereto. In addition, one of these joining methods may be used, or two or more types may be used in combination.

In addition, the adhesive may be any adhesive which has no cytotoxicity, and examples thereof include adhesives of synthetic compounds such as urethane adhesive, cyanoacrylate adhesive, polymethyl methacrylate (PMMA), calcium phosphate adhesive, and resin-based cement; adhesives of natural compounds such as fibrin glue, and gelatin glue, and the like.

In addition, the double-sided tape may be any tape which is not cytotoxic, and tapes used in medical applications or the like are suitably used. Specific examples thereof include tapes having a structure in which a pressure-sensitive adhesive layer is laminated on both sides of a support, and the pressure-sensitive adhesive layer is formed of a known pressure-sensitive adhesive which is rubber-based, acryl-based, urethane-based, silicone-based, or vinyl ether-based, or the like. More specific examples thereof include double-sided adhesive tape for skin application (product numbers: 1510, 1504 XL, 1524, and the like) manufactured by 3M Japan Ltd., double-sided adhesive tape for skin (product numbers: ST 502, ST 534, and the like) manufactured by Nitto Denko Corporation, double-sided medicinal tape (Product numbers: #1088, #1022, #1010, #809 SP, #414125, #1010 R, #1088 R, #8810 R, #2110 R, and the like) manufactured by Nichiban Medical Corp., thin foam material double-sided adhesive tape manufactured by DIC Corp., (product numbers: #84010, #84015, #84020, and the like), and the like.

Using double-sided adhesive tapes of different colors such as black and white (for example, #84010 WHITE, #84010 BLACK, and the like manufactured by DIC Corporation) on the top surface and bottom surface of the member 2, respectively, makes it possible to easily distinguish between the top surface side and the bottom surface side by visual observation in a case where the porous membrane 1 is transparent or translucent.

Next, it is possible to obtain the cell enclosure device 10 by carrying out sterilization using UV irradiation or the like, and adjusting the size of the porous membrane 1 or the member 2 as necessary.

In addition, in a case where the support 3 is provided as in the cell enclosure device 20 of the present embodiment shown in FIG. 2, the support 3 may be joined in advance to the porous membrane 1 or the member 2. Alternatively, the support 3 may be joined to the assembled cell enclosure device 20. The joining method may carry out the fixing using the same method as the joining method of the porous membrane and the member described above, or may carry out detachable attachment using a fastener or the like.

In addition, in a case of providing a tube as in the cell enclosure device 30 of the present embodiment shown in FIG. 3, the tube 4 may be inserted into the porous membrane 1 or the member 2 in advance. Alternatively, the tube 4 may be inserted into the assembled cell enclosure device 30. As a tube insertion method, for example, in a case where an indwelling needle is used as a tube, it is possible to insert the tube by inserting the indwelling needle into the cell enclosure device and then pulling out the inner needle.

### <<Method of Using Cell Enclosure Device>>

As described below, it is possible to use the cell enclosure device of the present embodiment for, for example, cell culturing, cell transporting, tissue-type chips, organ-type chips, organ-type chip systems, and the like.

In the present specification, "tissue" refers to a unit of a structure gathered in a pattern based on a certain lineage in which one type of stem cell is differentiated, and has a single role as a whole. For example, in epidermal keratinocytes, stem cells existing in the basal layer of the epidermis are differentiated into cells forming the granular layer through the spinous layer and are terminally differentiated to form a stratum corneum so as to exhibit a barrier function as the epidermis. Thus, constructing a multicellular structure including one type of cells derived from one cell lineage makes it possible for the tissue-type chip of the present embodiment to reproduce, for example, epithelial tissue, connective tissue, muscle tissue, nerve tissue, and the like.

In addition, in the present specification, an "organ" is formed of two or more types of tissues and has one function as a whole. Thus, constructing a multicellular structure including at least two types of cells having different cell lineages makes it possible for the organ-type chip of the present embodiment to reproduce, for example, a stomach, intestines, a liver, a kidney, and the like.

Furthermore, in the present specification, "organ system" refers to a group of two or more organs having similar functions and a group of two or more organs having a series of functions as a whole. Thus, in combination with a plurality of tissue-type chips or organ-type chips, it is possible for the organ-type chip system of the present embodiment to reproduce, for example, organ systems such as a digestive system, a cardiovascular system, a respiratory system, a urinary system, a reproductive system, an endocrine system, a sensory organ system, an exerciser system, and a nervous system. Living bodies maintain homeostasis by interactions between these organ systems. In the organ-type chip system of the present embodiment, since it is possible to combine a plurality of different organ-type chips of the organ system, it is also possible to analyze the interaction between different organs of the organ system. For example, in an organ-type chip system in which a small intestine-type chip, a liver-type chip, and a neural-type chip are connected in this order, in a case where a drug is added to the small intestine-type chip, the drug absorbed by the small intestine-type chip is metabolized by the liver-type chip, and it is possible to analyze the toxicity and the like exerted by the liver metabolites of the drug excreted by the liver-type chip on the neural-type chip.

### <Method for Culturing Cells>

The method for culturing cells of the present embodiment is a method using the cell enclosure device described above.

According to the culturing method of the present embodiment, it is possible to easily culture cells and construct a multicellular structure. In addition, it is possible to maintain cells for approximately 3 to 30 days, and to maintain cells for a longer period than in the related art. Furthermore, according to the culturing method of the present embodiment, it is possible to obtain the tissue-type chip described below.

A detailed description will be given below of the culturing method of the present embodiment.

First, a culture medium in which cells are suspended is prepared. Next, using an injection needle (including a winged needle, an indwelling needle, or the like) or the like, the suspension is injected into the cell enclosure device described above.

The injection needle may be injected by piercing the porous membrane, or may be injected by piercing the member. In a case of the injection needle piercing the porous membrane, using a cell enclosure device having a porous membrane having the material, content, and thickness provided as an exemplary example in the "Porous Membrane" described above makes use without breaking possible.

In addition, in a case where the material at the injection site (porous membrane or member) after injection of the culture medium in which the cells are suspended is high in hardness and low in elasticity, it is preferable to close the injection hole with a material having low hardness and high elasticity. On the other hand, in a case where the material at the injection site (porous membrane or member) is low in hardness and high in elasticity, it is preferable to close the injection hole with a material having high hardness and low elasticity.

More specifically, for example, in a case where the injection site is a member formed of silicone, the injection hole may be closed using a stainless-steel wire or the like, and, for example, in a case where the injection site is a member formed of polyacrylate, the injection hole may be closed with a thread formed of silicone, a wire formed of stainless steel, or the like.

Next, in the cell enclosure device into which the culture medium in which the cells are suspended is injected, culturing may be carried out in a gas phase and/or a liquid phase to construct a multicellular structure. The culturing in the gas phase may be performed, for example, by using a container such as an empty dish, and the culturing may be carried out within a time such that the cells do not dry and die. In addition, the culturing in the liquid phase may be performed using a container such as a dish including a culture medium, for example. In addition, the culturing in the gas phase and the liquid phase may be carried out, for example, by floating the cell enclosure device in a container such as a dish including the culture medium using the cell enclosure device having the support shown in FIG. 2.

Examples of the cells used in the culture method of the present embodiment include vertebrate cells such as mammalian cells, avian cells, reptile cells, amphibian cells, and fish cells; invertebrate cells such as insect cells, crustacean cells, molluscan cells, and protozoal cells; bacteria such as gram-positive bacteria (for example, Bacillus species), and gram-negative bacteria (for example, Escherichia coli or the like); yeasts, plant cells, small living organisms formed of single cells or a plurality of cells, and the like.

Examples of the small living organisms include unicellular organisms such as amoeba, paramecium, closterium, pinnularia, chlorella, euglena, and phacus; microcrustceans such as daphnia, artemia larvae, copepods, ostracoda, thecostraca larvae, phyllocarida shrimp larvae, peracarida shrimp larvae, and eucarida shrimp larvae; planaria (including regenerating planaria after fine cutting), terrestrial arthropod larvae, nemathelminthes, plant seeds (in particular, germinated seeds), callus, protoplast, marine microorganisms (for example, marine bacteria such as vibrio, pseudomonas, eromonas, alteromonas, flavobacterium, cytophaga, and flexibacter, algae such as cyanobacteria, cryptophytes, dinoflagellates, diatom, raphidophytes, golden algae, haptophytes, euglenophytes, prasinophyceae, and green algae, or the like), larval fish, larval shellfish, and the like, without being limited thereto.

For example, in a case where germinating seeds are cultured using the cell enclosure device of the present embodiment, since the top surface of the cell enclosure device has a hardness which is sufficient to allow germinated buds to penetrate and is formed of biodegradable material, germinated seeds placed in the device are able to be directly implanted in soil to allow plants to grow.

In the present specification, "biodegradable material" means a material having a property of being decomposed into inorganic matter by microorganisms or the like in soil or water.

Examples of vertebrate cells (in particular, mammalian cells) include reproductive cells (sperm, eggs, or the like), somatic cells, stem cells, and progenitor cells forming a living body, cancer cells separated from a living body, cells (cell lines) separated from a living body and stably maintained outside by being immortalized, cells separated from a living body and artificially genetically modified, cells separated from a living body and with the nuclei artificially exchanged, and the like, without being limited thereto. In addition, aggregates of cells (spheroids) of these cells may also be used. In addition, a small tissue piece separated from normal tissue or cancer tissue of a living body may be used as it is in the same manner as a multicellular aggregate.

Examples of somatic cells forming a living body include cells collected from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood, heart, eye, brain, and nerve tissue, without being limited thereto. More specifically, examples of somatic cells include fibroblasts, bone marrow cells, immune cells (for example, B lymphocytes, T lymphocytes, neutrophils, macrophages, monocytes, or the like), red blood cells, platelets, osteocytes, pericytes, dendritic cells, epidermal keratinocytes (keratinocytes), adipocytes, mesenchymal cells, epithelial cells, epidermal cells, endothelial cells, vascular endothelial cells, lymphatic endothelial cells, hepatocytes, islet cells (for example, α cells, β cells, δ cells, ε cells, PP cells, or the like), chondrocytes, cumulus cells, glial cells, neural cells (neurons), oligodendrocytes, microglia, astrocytes, cardiomyocytes, esophageal cells, muscle cells (for example, smooth muscle cells, skeletal muscle cells, or the like), melanocytes, mononuclear cells, and the like, without being limited thereto.

A stem cell is a cell which combines the ability to replicate itself and the ability to differentiate into cells of a plurality of other lines. Examples of stem cells include embryonic stem cells (ES cells), embryonic tumor stem cells, embryonic reproductive stem cells, induced pluripotent stem cells (iPS cells), neural stem cells, hematopoietic stem cells, mesenchymal stem cells, hepatic stem cells, pancreatic stem cells, muscle stem cells, reproductive stem cells, intestinal stem cells, cancer stem cells, hair follicle stem cells, and the like, without being limited thereto.

A progenitor cell is a cell in the stage of being differentiated from a stem cell into a specific somatic cell or a reproductive cell.

A cancer cell is a cell derived from a somatic cell and acquiring infinite proliferative capacity and is a malignant neoplasm which invades or causes metastasis in the surrounding tissue. Examples of cancers from which cancer cells are derived include breast cancer (for example, invasive ductal breast cancer, non-invasive ductal breast cancer, inflammatory breast cancer, and the like), prostate cancer (for example, hormone-dependent prostate cancer, hormone-independent prostate cancer, and the like), pancreatic cancer (for example, pancreatic duct cancer, and the like), stomach cancer (for example, papillary adenocarcinoma, mucinous adenocarcinoma, adenosquamous cancer, and the like), lung cancer (for example, non-small cell lung cancer, small cell lung cancer, malignant mesothelioma, and the like), colon cancer (for example, gastrointestinal stromal tumors, and the like), rectal cancer (for example, gastrointestinal stromal tumors, and the like), colorectal cancer (for example, familial colorectal cancer, hereditary non-polyposis colon cancer, gastrointestinal stromal tumors, and the like), small bowel cancer (for example, non-Hodgkin's lymphoma, gastrointestinal stromal tumors, and the like), esophageal cancer, duodenal cancer, tongue cancer, pharyngeal cancer (for example, nasopharyngeal cancer, oropharyngeal cancer, hypopharyngeal cancer, and the like), head and neck cancer, salivary gland cancer, brain tumors (for example, pineal gland astrocytoma, pilocytic astrocytoma, diffuse astrocytoma, anaplastic astrocytoma, and the like), neurinoma, liver cancer (for example, primary liver cancer, extrahepatic bile duct cancer, and the like), kidney cancer (for example, renal cell cancer, transitional epithelial cancer of renal pelvis and ureter, and the like), gallbladder cancer, pancreatic cancer, endometrial cancer, cervical cancer, ovarian cancer (for example, epithelial ovarian cancer, extragonadal germ cell tumors, ovarian germ cell tumors, low grade ovarian tumors, and the like), bladder cancer, urethral cancer, skin cancer (for example, intraocular (ocular) melanoma, Merkel cell cancer, and the like), angioma, malignant lymphoma (for example, reticulosarcoma, lymphosarcoma, Hodgkin's disease, and the like), melanoma (malignant melanoma), thyroid cancer (for example, medullary cancer of the thyroid, and the like), parathyroid cancer, nasal cancer, paranasal sinus cancer, bone tumors (for example, osteosarcoma, Ewing's tumor, uterine sarcoma, soft tissue sarcoma, and the like), metastatic medulloblastoma, angiofibroma, protruding dermal fibrosarcoma, retinal sarcoma, penile cancer, testicular tumors, pediatric solid cancer (for example, Wilms tumor, pediatric renal tumors, and the like), Kaposi's sarcoma, Kaposi's sarcoma caused by AIDS, maxillary sinus tumors, fibrous histiocytoma, leiomyosarcoma, rhabdomyosarcoma, chronic myeloproliferative disease, leukemia (for example, acute myelogenous leukemia, acute lymphoblastic leukemia, and the like) and the like, without being limited thereto.

In addition, in the present specification, the Chinese character for "cancer" is used to indicate a diagnosis name and the Japanese characters for "cancer" are used to represent a generic term for a malignant neoplasm.

A cell line is a cell which acquired infinite proliferative capacity due to artificial manipulation in vitro. Examples of cell lines include HCT116, Huh7, HEK293 (human embryonic kidney cells), HeLa (human cervical cancer cell line), HepG2 (human liver cancer cell line), UT7/TPO (human leukemia cell line), CHO (Chinese hamster ovary cell line), MDCK, MDBK, BHK, C-33A, HT-29, AE-1, 3D9, Ns 0/1, Jurkat, NIH3T3, PC12, S2, Sf9, Sf21, High Five, Vero, and the like, without being limited thereto.

The culture medium of animal cells used in the culturing method of the present embodiment may be a basic culture medium including components necessary for the cell survival and growth (inorganic salts, carbohydrates, hormones, essential amino acids, non-essential amino acids, vitamins) and the like, and is able to be appropriately selected according to the type of cells. Examples of the culture medium include Dulbecco's Modified Eagle's Medium (DMEM), Minimum Essential Medium (MEM), RPMI-1640, Basal Medium Eagle (BME), Dulbecco's Modified Eagle's Medium: Nutrient Mixture F-12 (DMEM/F-12), Glasgow Minimum Essential Medium (Glasgow MEM), and the like, without being limited thereto.

In addition, for a culture medium of bacteria, yeasts, plant cells, and small living organisms formed from single cells or a plurality of cells, a culture medium having a composition suitable for growth in each case may be prepared.

In addition, in the culturing method of the present embodiment, a component derived from an extracellular matrix, a physiologically active substance, and the like may be mixed and injected into a culture medium in which cells are suspended.

Examples of the component derived from an extracellular matrix include the same examples provided as exemplary examples of the "Porous Membrane" described above.

Examples of physiologically active substances include cell growth factors, differentiation inducing factors, cell adhesion factors, and the like, without being limited thereto. For example, by including a differentiation inducing factor, in a case where the cells to be injected are stem cells, precursor cells, or the like, it is possible to induce differentiation of the stem cells or the precursor cells and to construct a multicellular structure reproducing a desired tissue.

In addition, in the culturing method of the present embodiment, the culture medium in which the cells are suspended may be injected so as to fill the capacity of the cell enclosure device, or an amount less than the capacity of the cell enclosure device may be injected. For example, in a case where the cell enclosure device has a structure in which a porous membrane is provided on the top surface and the bottom surface as shown in FIG. 1 and the material of the porous membrane is collagen, a culture medium in which the cells are suspended is injected with an injection needle or the like in an amount less than the capacity of the cell enclosure device and the needle or the like is pulled out. Due to this, the top surface and the bottom surface of the cell enclosure device are depressed under reduced pressure, and the cells are sandwiched between the porous membrane on the top surface and the porous membrane on the bottom surface, and it is possible to perform sandwich culturing using collagen.

In the culturing method of the present embodiment, it is possible to appropriately select the culture conditions of animal cells according to the type of cells to be cultured.

The culturing temperature may be, for example, 25°C or more and 40°C or less, for example, 30°C or more and 39°C or less, and, for example, 35°C or more and 39°C or less.

In addition, the culture environment may be under a condition of, for example, approximately 5% CO₂.

It is possible to appropriately select the culturing time according to the type of cells, the number of cells and the like, and the culturing time may be, for example, 3 days or more and 30 days or less, for example, 5 days or more and 20 days or less, and, for example, 7 days or more and 15 days or less.

In addition, for the culturing conditions of bacteria, yeasts, plant cells, and small living organisms formed of single cells or a plurality of cells thereof, the environment and time may be set to be suitable for growth in each case.

### <Cell Transportation Method>

The cell transportation method of the present embodiment is a method using the cell enclosure device described above.

According to the transportation method of the present embodiment, it is possible to transport cells safely and reliably and also to handle long transportation periods.

A detailed description will be given below of the transportation method of the present embodiment.

First, a culture medium in which cells are suspended is prepared. Next, using an injection needle (including a winged needle, an indwelling needle, or the like) or the like, a culture medium in which the cells are suspended is injected into the cell enclosure device described above.

Examples of the cells or small living organisms used in the transportation method of the present embodiment include the same examples as those listed as exemplary examples in the "Method for Culturing Cells" described above. In addition, examples of the culture medium used in the transportation method of the present embodiment include the same examples provided as exemplary examples of the above "Method for Culturing Cells".

In addition, in the cell transportation method of the present embodiment, in a case where the cells are animal cells, a component derived from an extracellular matrix, physiologically active substances, and the like may be mixed and injected into a culture medium in which cells are suspended.

Examples of components derived from an extracellular matrix include the same examples provided as exemplary examples of the "Porous Membrane" described above.

In addition, examples of the physiologically active substance include the same examples provided as exemplary examples of the "Method for Culturing Cells" described above.

The cells enclosed in the cell enclosure device may be in the process of constructing a multicellular structure or may be after the multicellular structure was constructed. Of the two, since it is possible to use the cells immediately for an in vitro test system or living body transplantation, the cells enclosed in the cell enclosure device in the transportation method of the present embodiment are preferably after the multicellular structure was constructed.

Next, the cell enclosure device (a tissue-type chip described below or an organ-type chip described below) in which cells are enclosed is enclosed in an openable and closable sealed container including a culture medium and transported.

The sealed container in the transportation method of the present embodiment is not particularly limited as long as the sealed container is openable and closable. Examples of the sealed container include a conical tube with a screw cap, a flask for cell culturing with a screw cap, and the like, without being limited thereto.

It is possible to appropriately select the transporting conditions depending on the type of cells or small living organisms to be transported.

The temperature during transportation may be, for example, 4°C or more and 40°C or less, for example, 10°C or more and 39°C or less, and, for example, 18°C or more and 37°C or less.

In addition, in the environment during transportation, in a case where the cells are animal cells, the cell enclosure device in which the cells are enclosed may be in a state of being enclosed in a sealed container filled up to the full capacity with the culture medium. Alternatively, the cell enclosure device in which the cells are enclosed may be in a state of being enclosed in a sealed container partially filled with the culture medium, and the gas portion in the sealed container may be under a condition, for example, of being air containing approximately 5% CO₂.

It is possible to appropriately select the transporting time depending on the type of cells, the number of cells, and the like, and the transporting time may be, for example, 1 hour or more and 30 days or less, for example, 1 day or more and 20 days or less, and, for example, 2 days or more and 7 days or less.

The cell enclosure device (the tissue-type chip described below or the organ-type chip described below) in which the cells are enclosed may be used as it is for an in vitro test system or living body transplantation, or may be used by destroying the cell enclosure device and taking out the enclosed cells for the purpose of proliferation culturing or for the purpose of transplantation, or the like.

### <Tissue-Type Chip>

The tissue-type chip of the present embodiment is provided with the cell enclosure device described above in which one type of cell (in particular, animal cells) is enclosed.

The tissue-type chip of the present embodiment does not need to construct a culture model from nothing and use is possible as a substitute for the culture models or animal experiments of the related art in the screening of candidate drugs for various diseases or in evaluation test systems for the pharmacokinetics and toxicity of chemical substances, including candidate drugs, with respect to normal tissues.

Furthermore, the culture models or regenerated tissue for transplantation of the related art are subject to a time restriction and have to be used immediately after construction, while it is possible to culture the tissue-type chip of the present embodiment for long periods.

In addition, in the tissue-type chip of the present embodiment, in a case where the cell enclosure device is formed of a material having biocompatibility, it is possible to expect uses for regenerative medicine as a medical cell transplantation device.

Examples of cells enclosed in the tissue-type chip of the present embodiment include the same cells as the exemplary examples in "Method for Culturing Cells" described above. In addition, the type of enclosed cells may be any type appropriately selected according to the type of tissue to be constructed.

In addition, the cells enclosed in the tissue-type chip of the present embodiment may be in the process of constructing a multicellular structure or may be after the multicellular structure was constructed. It is possible for the tissue-type chip of the present embodiment to be cultured for a long period of approximately 3 to 21 days even after the enclosed cells constructed the multicellular structure.

The density of cells enclosed in the tissue-type chip of the present embodiment varies depending on the type of tissue to be constructed, but is preferably 2.0 × 10³ cells/mL or more and 1.0 × 10⁹ cells/mL or less, and more preferably 2.0 × 10⁵ cells/mL or more and 1.0 × 10⁷ cells/mL or less.

When the cell density is within the range described above, it is possible to obtain a tissue-type chip having a cell density closer to that of a living tissue.

It is possible to produce the tissue-type chip of the present embodiment using the method described in the "Method for Culturing Cells". In addition, the maintenance conditions of the tissue-type chip after production may be the same as the culturing conditions described in the above "Method for Culturing Cells". In addition, the interior of the tissue-type chip may include a culture medium or a gas such as air, or may not include a culture medium or a gas such as air. In a case where the tissue-type chip does not include a culture medium or a gas such as air, cells, or cells and components derived from an extracellular matrix are closely adhered together and a multicellular structure with a configuration closer to tissue in a living body is constructed.

### <Organ-Type Chip>

The organ-type chip of the present embodiment is provided with the cell enclosure device described above in which at least two types of cells (in particular, animal cells) are enclosed.

The organ-type chip of the present embodiment does not need to construct a culture model from nothing, and use is possible as a substitute for the culture models or animal experiments of the related art in the screening of candidate drugs for various diseases or in evaluation test systems for pharmacokinetics and toxicity of chemical substances, including candidate drugs, with respect to normal organs.

Furthermore, the culture model or the regenerated tissue for transplantation of the related art are subject to a time restriction and have to be used immediately after construction, while it is possible to culture the organ-type chip of the present embodiment for long periods.

In addition, in the organ-type chip of the present embodiment, in a case where the cell enclosure device is formed of a material having biocompatibility, it is possible to expect uses for regenerative medicine as a medical cell transplantation device.

Examples of the cells enclosed in the organ-type chip of the present embodiment include the same cells as the exemplary examples in the "Method for Culturing Cells" described above. In addition, as long as at least two types of cells are enclosed, the type of enclosed cells may be any type which is appropriately selected according to the type of the organ to be constructed.

In addition, the cells enclosed in the organ-type chip of the present embodiment may be in the process of constructing a multicellular structure or may be after the multicellular structure was constructed. It is possible for the organ-type chip of the present embodiment to be cultured for a long period of approximately 3 to 21 days even after the enclosed cells have constructed the multicellular structure.

In addition, for example, in the organ-type chip of the present embodiment, a multicellular structure (that is, epithelial tissue) formed of epithelial cells (for example, epidermal keratinocytes and the like) is constructed on the top surface on the inner side of the cell enclosure device and a multicellular structure (that is, mesenchymal tissue) formed of mesenchymal cells (for example, dermal fibroblasts and the like) is constructed on the bottom surface on the inner side, making it possible to easily reproduce the exchanging of substances between tissues in cell enclosure device.

The density of cells enclosed in the organ-type chip of the present embodiment varies depending on the type of the organ to be constructed, but is preferably 2.0 × 10³ cells/mL or more and 1.0 × 10⁹ cells/mL or less, and more preferably 2.0 × 10⁵ cells/mL or more and 1.0 × 10⁷ cells/mL or less.

When the cell density is within the range described above, it is possible to obtain an organ-type chip having a cell density closer to that of living tissue.

It is possible to produce the organ-type chip of the present embodiment using the method described in "Method for Culturing Cells" described above. In addition, the maintenance conditions of the organ-type chip after production may be the same as the culturing conditions described in "Method for Culturing Cells" described above. In addition, the interior of the organ-type chip may include a culture medium or a gas such as air, or may not include a culture medium or a gas such as air. In a case where organ-type chips do not include a culture medium or a gas such as air, cells, or cells and components derived from an extracellular matrix are closely adhered together and a multicellular structure with a configuration closer to organs in a living body is constructed.

### <Kit for Providing Multicellular Structure>

The kit of the present embodiment is a kit for providing a multicellular structure, and is provided with an openable and closable sealed container including the tissue-type chip described above or the organ-type chip described above and a culture medium.

The kit of the present embodiment does not need to construct a culture model from nothing, and use is possible as a substitute for the culture models or animal experiments of the related art in the screening of candidate drugs for various diseases or in evaluation test systems for the pharmacokinetics and toxicity of chemical substances, including candidate drugs, with respect to normal tissues or organs.

Furthermore, the culture model or the regenerated tissue for transplantation of the related art are subject to a time restriction and have to be used immediately after construction, while it is possible to culture the kit of the present embodiment for long periods.

The cells enclosed in the tissue-type chip or the organ-type chip in the kit of the present embodiment may be in the process of constructing a multicellular structure or may be after the multicellular structure was constructed. Among these, since it is possible to use the cells immediately for an in vitro test system or living body transplantation, the cells enclosed in the tissue-type chip or the organ-type chip in the kit of the present embodiment are preferably after the multicellular structure was constructed.

Examples of the sealed container in the kit of the present embodiment include the same containers provided as exemplary examples in the "Cell Transportation Method" described above.

Any material which has liquid-tightness may be used as the material of the sealed container. In addition, the sealed container may have air permeability or may not have air permeability. More specifically, examples of the material of the sealed container are the same as the exemplary examples provided for the "Member" described above. Among these, as the material of the sealed container, plastic is preferable due to being hard to break and lightweight.

It is possible to appropriately select the culture medium in the kit of the present embodiment depending on the type of cells enclosed in the tissue-type chip or the organ-type chip, specific examples thereof include the exemplary examples provided in "Method for Culturing Cells" described above.

In addition, in the kit of the present embodiment, the culture medium is preferably included to the full capacity of the sealed container. Injecting and sealing the culture medium into the sealed container at the full capacity thereof prevents drying of tissue-type chips or organ-type chips and makes it possible to safely carry the tissue-type chips or organ-type chips.

In the kit of the present embodiment, the number of tissue-type chips or organ-type chips included in the sealed container may be one or may be two or more. In a case of including two or more, tissue-type chips or organ-type chips in which the same type of multicellular structure is constructed are preferable.

The kit of the present embodiment may be further provided with a culture medium separate from the culture medium included in the sealed container. The culture medium may be the same type as the culture medium included in the sealed container or may be another type. By separately providing a culture medium, use is possible as an exchange culture medium for culturing tissue-type chips or organ-type chips until the kit of the present embodiment is used in an in vitro test system, living body transplantation, or the like.

### <Organ-Type Chip System>

The organ-type chip system of the present embodiment is provided with at least two of the tissue-type chips or the organ-type chips described above, and the tissue-type chips or the organ-type chips are connected while maintaining the cell enclosure property.

The organ-type chip system of the present embodiment does not need to construct a culture model from nothing, and use is possible as a substitute for the culture models or animal experiments of the related art in the screening of candidate drugs for various diseases or in evaluation tests or the like for the pharmacokinetics and toxicity of chemical substances, including candidate drugs, with respect to a plurality of normal tissues or organs.

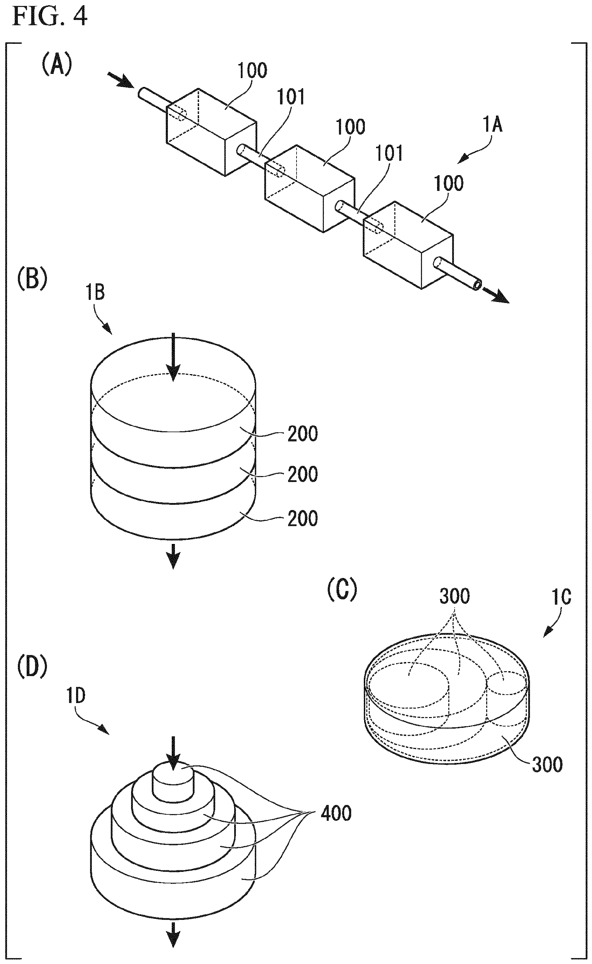

FIG. 4(A) is a perspective view schematically showing the organ-type chip system according to the first embodiment of the present invention.

An organ-type chip system 1A shown here has a structure in which three tissue-type chips 100 are connected via a tube 101, respectively.

For example, allowing the culture medium to flow from the left arrow direction to the right arrow direction makes it possible to culture the three tissue-type chips 100 in a connected state. In addition, for example, allowing candidate drugs for various diseases to flow in the direction of the arrow on the right side from the direction of the arrow on the left side makes it possible to verify the drug efficacy against diseases, the metabolic pathways of the drug and the metabolites thereof, the cytotoxicity, and the like.

The tissue-type chip 100 shown in FIG. 4(A) is the same as in "Tissue-type Chip" described above. It is possible to appropriately select the type of cells (not shown) forming the multicellular structure constructed in the tissue-type chip 100 depending on the desired type of organ or organ system.

In addition, the tube 101 shown in FIG. 4(A) is the same as the tube 4 in FIG. 3, and has the same configuration as described in "Tube" described above.

FIG. 4(B) is a perspective view schematically showing the organ-type chip system according to the second embodiment of the present invention.

The organ-type chip system 1B shown here has a structure in which three tissue-type chips 200 of the same size are stacked. At this time, at least the top surface and the bottom surface of each tissue-type chip 200 are porous membranes.

For example, allowing the culture medium to flow from the direction of the upper arrow to the direction of the lower arrow makes it possible to culture the three tissue-type chips 200 in a stacked state. In addition, for example, allowing candidate drugs for various diseases to flow in the direction of the lower arrow from the direction of the upper arrow makes it possible to verify the drug efficacy against diseases, the metabolic pathways of the drug and the metabolites thereof, the cytotoxicity, and the like.

FIG. 4(C) is a perspective view schematically showing the organ-type chip system according to the third embodiment of the present invention.

The organ-type chip system 1C shown here has four tissue-type chips 300 of different sizes and has a structure in which a small tissue-type chip 300 is enclosed in the largest tissue-type chip 300. At this time, at least the top surface and the bottom surface of the largest tissue-type chip 300 are porous membranes, and the tissue-type chip 300 enclosed in the largest tissue-type chip 300 is a whole surface porous membrane.

For example, it is possible to carry out the culturing by placing the organ-type chip system 1C in a container such as a dish including a culture medium. In addition, for example, placing the organ-type chip system 1C in a container such as a dish including a candidate drug for various diseases makes it possible to verify the drug efficacy against diseases, the metabolic pathways of the drug and the metabolites thereof, the cytotoxicity, and the like.

FIG. 4(D) is a perspective view schematically showing the organ-type chip system according to the fourth embodiment of the present invention.

The organ-type chip system 1D shown here is a structure in which four tissue-type chips 400 having different sizes are stacked from the bottom in decreasing order of size. At this time, at least the top surface and the bottom surface of each tissue-type chip 400 are porous membranes.

For example, allowing the culture medium to flow from the direction of the upper arrow to the direction of the lower arrow makes it possible to culture the four tissue-type chips 400 in a stacked state. In addition, for example, allowing the candidate drug for various diseases to flow in the lower arrow direction from the direction of the upper arrow makes it possible to verify the drug efficacy against diseases, the metabolic pathways of the drug and the metabolites thereof, the cytotoxicity, and the like.

The organ-type chip system according to the present embodiment is not limited to FIG 4(A) to FIG. 4(D), but parts of the configuration shown in FIG. 4(A) to FIG. 4(D) may be changed or removed or other configurations may be added to the embodiments previously described, within a range in which the effect of the organ-type chip system of the present embodiment is not impaired.

For example, in FIG. 4(A) to FIG. 4(D), a case where a tissue-type chip is provided was provided as an exemplary example, but an organ type may be provided at least in part.

For example, in the organ-type chip system shown in FIG. 4(A), each tube may be provided with an openable and closable device such as a plug or a valve.

In addition, in the organ-type chip system shown in FIG. 4(B) and FIG. 4(D), each tissue-type chip may have a support and furthermore, in order to fix each tissue-type chip, the outer periphery of the top surface and the bottom surface may be fixed with an adhesive or the like.

In addition, in the organ-type chip system of the present embodiment, it is possible to arbitrarily adjust the size and shape of each configuration (tissue-type chip, tube, or the like) according to the purpose.

The organ-type chip system of the present embodiment itself is able to reproduce organs such as the liver, stomach, intestines, and the like. Furthermore, combining a plurality of the organ-type chip systems of the present embodiment makes it possible to reproduce organ systems such as the digestive system, the cardiovascular system, the respiratory system, the urinary system, the reproductive system, the endocrine system, the sensory organ system, the exerciser system, and the nervous system.

### Examples

A description will be given below of the present invention with reference to Examples, but the present invention is not limited thereto.

### [Production Example 1] Production of Cell Enclosure Device (with Support) 1

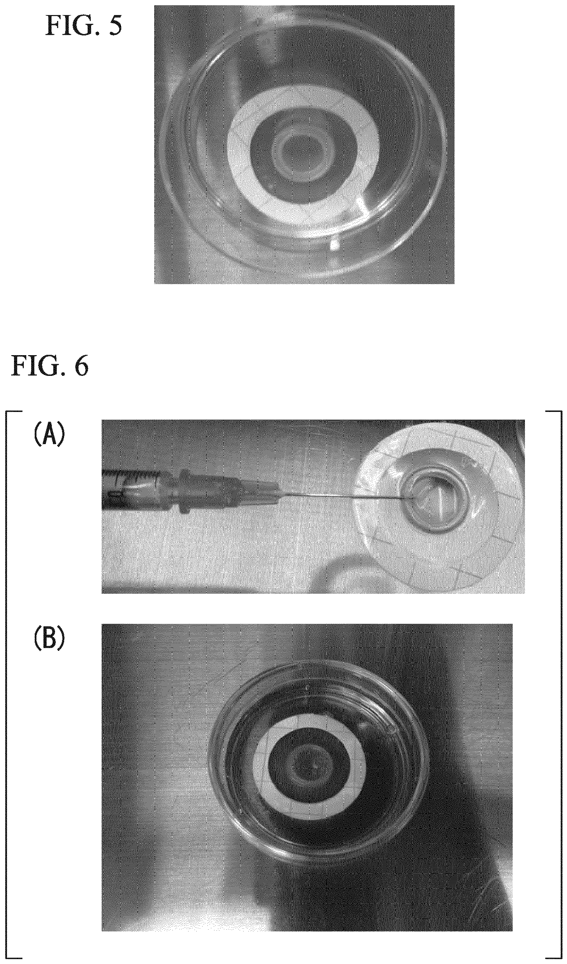

(1) First, a native collagen Vitrigel (registered trademark) membrane with a ring-shaped nylon membrane support (content per unit area of native collagen: 0.5 mg/cm²) (may be simply referred to as "collagen Vitrigel (registered trademark) membrane") was prepared according to a known method (reference: Japanese Unexamined Patent Application, First Publication No. 2015-35978).
(2) Next, the prepared collagen Vitrigel (registered trademark) membrane was stretched on a vinyl sheet, and a silicon ring (inner diameter 9.8 mm × thickness 1.9 mm) was placed thereon.
(3) Next, 0.4 mL of a 0.25% collagen sol solution is added around the silicon ring on the collagen Vitrigel (registered trademark) membrane and one more collagen Vitrigel (registered trademark) membrane is overlapped thereon such that the silicon ring is sandwiched in the middle of two collagen Vitrigel (registered trademark) membranes.
   According to a known method (reference: Japanese Unexamined Patent Application, First Publication No. 2015-203018), a 0.25% collagen sol solution used as an adhesive was prepared by mixing equal amounts of a collagen acidic solution I-AC for cell culturing (manufactured by Koken Co., Ltd.) and DMEM containing 10% fetal bovine serum (FBS), 20 mM HEPES, 100 units/mL penicillin, and 100 µg/mL streptomycin as a culture medium (may be simply referred to as "culture medium").
(4) Next, the result was dried (vitrified) using a clean air dryer in an incubator at 10°C and 40% humidity.
(5) Next, a one size smaller silicon ring (7.8 mm in inner diameter × 1.9 mm in thickness) was wrapped in aluminum foil and overlapped on a silicon ring sandwiched between dried collagen Vitrigel (registered trademark) membranes, and the collagen Vitrigel (registered trademark) membrane dried bodies inside the silicon ring were masked and irradiated with ultraviolet rays (may be referred to below as "UV") (total UV irradiation amount per unit area: 400 mJ/cm²).
(6) The collagen Vitrigel (registered trademark) membrane dried bodies after UV irradiation were inverted and the collagen Vitrigel (registered trademark) membrane dried bodies inside the silicon ring were masked and irradiated with UV (total UV irradiation amount per unit area: 400 mJ/cm²) in the same manner.
(7) Vitrification was promoted in a dish or the like to produce a cell enclosure device (refer to FIG. 5).

It was confirmed that the culture medium was injected into the produced cell enclosure device using a 1 mL tuberculin needle-equipped syringe and the culture medium was able to be retained without leaking from the inside of the device even after the needle was removed.

### [Example 1] Preparation of Hepatic Tissue-Type Chip Using HepG2 Cell of Human Liver Cancer Cell Line 1

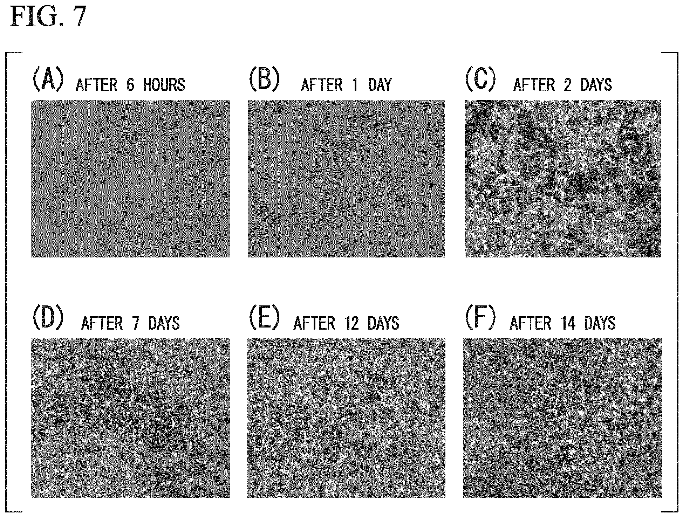

(1) Pre-cultured HepG2 cells (purchased from RIKEN BioResource Center, RCB 1648) were collected and mixed with the culture medium so as to be 2.0 × 10⁵ cells/mL to prepare a suspension of HepG2 cells.
(2) Next, a suspension of HepG2 cells was filled in a 1 mL syringe with a tuberculin needle. Next, the tip of a tuberculin needle was slightly injected into the inside of the silicon ring from the outer peripheral portion of the silicon ring, the cell enclosure device prepared in Production Example 1. Next, 160 µL of a suspension of HepG2 cells was injected into the cell enclosure device (refer to FIG. 6(A)).
(3) Next, 5 mL of the culture medium was poured into a dish (60 mm in diameter), the device enclosing HepG2 cells in (2) was floated on the culture medium, and culturing in the gas phase and the liquid phase was started (refer to FIG. 6(B)).
(4) Subsequently, the culture medium was exchanged every other day, HepG2 cells enclosed in the cell enclosure device were observed and photographed over time using a phase contrast microscope after 6 hours from the start of the culture and on days 1, 2, 7, 12, and 14 (refer to FIG. 7(A) to FIG. 7(F)).

From FIG. 7(A) to FIG. 7(F), it was confirmed that HepG2 cells proliferated over time and that a bile canaliculus-like structure was formed on day 2 of culturing.

### [Example 2] Metabolism of Model Drug by HepG2 Cell Hepatic Tissue-Type Chip and Excretion and Accumulation of Metabolites to Bile Canaliculus-Like Structure 1

(1) A culture medium containing fluorescein diacetate (FD) as a model drug at a concentration of 250 µg/mL was prepared, and 5 mL thereof was poured into a dish (60 mm in diameter).
(2) Next, the hepatic tissue-type chip on day 7 of culturing produced using HepG2 cells in Example 1 was immersed in the FD-containing culture medium prepared in (1) and cultured for 1 hour, such that the FD was taken into the cells.
(3) Next, the chip was transferred into a new dish (diameter: 60 mm), into which 5 mL of Hank's Balanced Salt Solution (HBSS) was poured, and washed. After performing this operation twice, it was confirmed by observation with a fluorescence microscope that green fluorescence (excitation wavelength: 490 nm, fluorescence wavelength: 514 nm) of fluorescein metabolized by HepG2 cells was uniformly distributed in the cytoplasm.
   Although FD does not generate fluorescence as it is, green fluorescence (excitation wavelength: 490 nm, fluorescence wavelength: 514 nm) of fluorescein is observed due to the ester bond cleavage caused by intracellular esterase activity.
(4) Next, the hepatic tissue-type chip was transferred into a new dish (diameter 60 mm) into which 5 mL of a fresh culture medium was poured, immersed in the culture medium, and cultured for 1 hour. The discharging of fluorescein in the cells after 1 hour was confirmed by observation with a phase contrast microscope and a fluorescence microscope (refer to FIG. 8(A) to FIG. 8(C)).

From FIG. 8(A) to FIG. 8(C), it was confirmed that fluorescein distributed in the cytoplasm was excreted and accumulated in the bile canaliculus-like structure.

### [Production Example 2] Production of Cell Enclosure Device (Arbitrary Shape) 2

(1) After a silicone membrane having a thickness of 3 mm was cut out into a ring shape (inner diameter: 11 mm, outer diameter: 20 mm) (refer to FIG. 9(A)), holes were opened in two places in the side surface (refer to FIG. 9(B)) by passing through a stainless-steel pipe (inner diameter: 0.9 mm, outer diameter: 1.26 mm) so as to penetrate the inside and outside of the ring shape at approximately the center portion in the thickness of the silicone membrane.
(2) Next, the collagen Vitrigel (registered trademark) membrane dried body was adhered with a urethane-based adhesive to the top surface and bottom surface by a known method (reference: Japanese Unexamined Patent Application, First Publication No. 2012-115262) such that the ring-shaped silicone membrane produced in (1) was a member of the side surface (refer to FIG. 9(C)). Next, a gel loading chip was passed through the two holes on the side surface to produce a cell enclosure device (refer to FIG. 9(D).).
   Here, silicone membranes having various thicknesses are commercially available, and it is possible to produce a cell enclosure device having an arbitrary shape by producing a member of a side surface by cutting the silicone membranes into a desired shape and then pasting the collagen Vitrigel (registered trademark) membrane dried body to the top surface and bottom surface of the member of the side surface.
(3) Next, it was confirmed that it is possible to inject an appropriate amount of culture medium into the device by connecting a syringe to the gel loading chip of the cell enclosure device produced in (2) (refer to FIG. 9(E) and FIG. 9(F)).
   In addition, it was confirmed that it is possible to pass the culture medium from one cell enclosure device to another cell enclosure device by connecting two cell enclosure devices with a tube at the chip portion of the gel loading chip (refer to FIG. 9(G)).
(4) Next, it was confirmed that, after detaching the gel loading chip from the cell enclosure device into which an appropriate amount of culture medium was injected and then plugging the hole with a toothpick, the culture medium does not leak out from the inside of the cell enclosure device (refer to FIG. 9(H)).

In the present Example, a wooden toothpick is used as a plug, but a plug formed of a plastic such as acrylic or stainless steel is preferable for culturing.

From the above results, it was confirmed that it is possible to culture cells for a long period using the cell enclosure device of the present embodiment, and to construct a multicellular structure.

### [Production Example 3] Production of Cell Enclosure Device (No Support) 3

(1) Using a silicon ring (inner diameter: 7.8 mm, thickness: 1.9 mm) which is slightly smaller than the silicon ring used in Production Example 1, a cell enclosure device was produced using the same method as in Production Example 1. Next, the ring-shaped nylon membrane was removed and the collagen Vitrigel (registered trademark) membrane dried body protruding to the outer peripheral portion was folded together with the collagen sol around the silicon ring and dried.
(2) Next, the collagen Vitrigel (registered trademark) membrane dried body on the inner side of the silicon ring was masked, and both sides were adhered together and fixed with UV irradiation (single side irradiation amount: 400 mJ/cm², total irradiation amount: 800 mJ/cm²) to produce a cell enclosure device with no support.

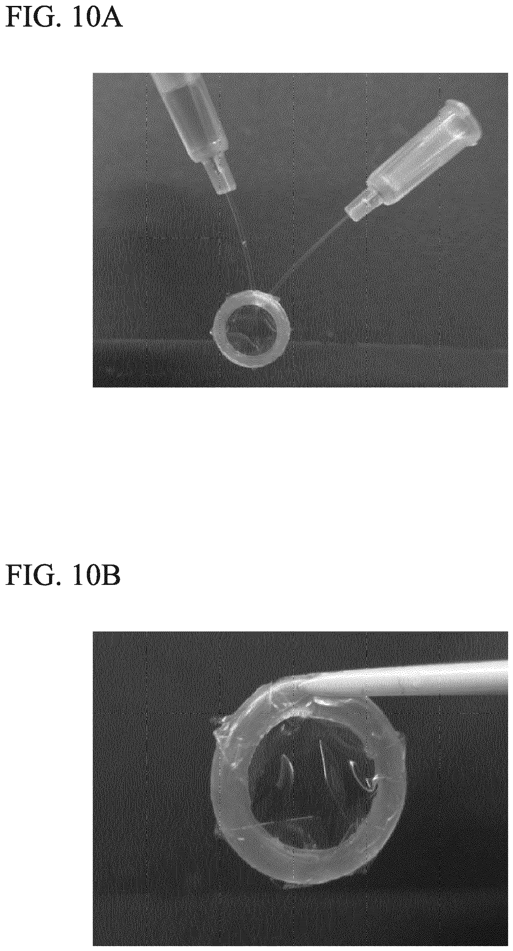

For the obtained cell enclosure device with no support, it was confirmed that it is possible to safely enclose the suspension of HepG2 cells therein using an indwelling needle (refer to FIG. 10A and FIG. 10B).

### [Example 3] Production of Hepatic Tissue-Type Chip Using HepG2 Cells of Human Liver Cancer Cell Line 2

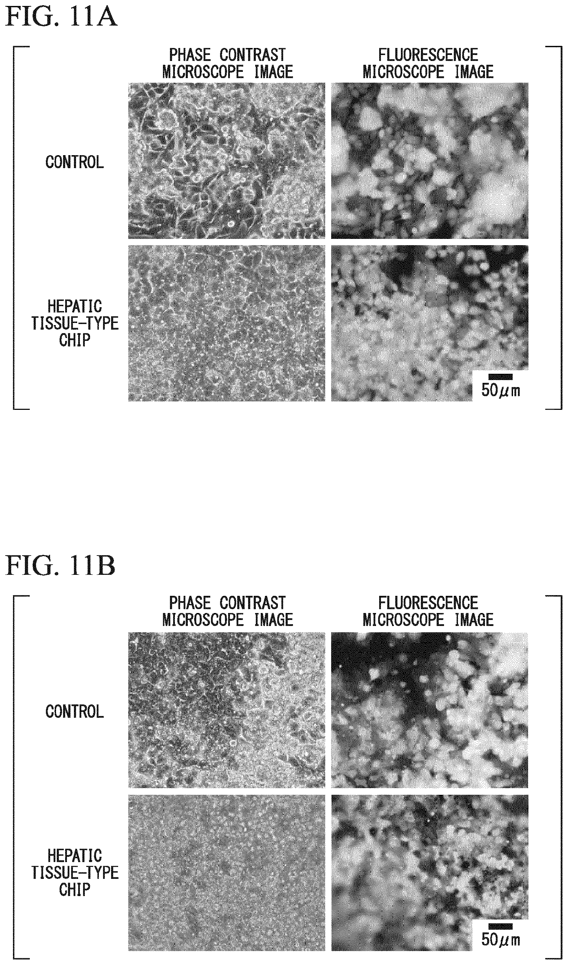

(1) Pre-cultured HepG2 cells (purchased from RIKEN BioResource Center, RCB 1648) were collected and mixed with a culture medium to be 2.4 × 10⁵ cells/mL to prepare a suspension of HepG2 cells.
(2) An indwelling needle was punctured from the silicon ring wall surface of the cell enclosure device obtained in Production Example 3 and the inner needle was removed, resulting that an indwelling needle catheter was attached thereto. Next, a suspension (2.4 × 10⁵ cells/mL) of 100 µL of HepG2 cells was injected using this indwelling needle catheter to produce a hepatic tissue-type chip. Since the inner bottom surface area of the silicon ring was 0.48 cm², the initial seeding density was 5.0 × 10⁴ cells/cm².
(3) 1.0 mL of the culture medium was poured into wells of a 24-well plate, the hepatic tissue-type chip produced in (2) was immersed in the culture medium and culturing was started. As a control, a suspension of 1.0 mL of HepG2 cells prepared to have an initial seeding density of 5.0 × 10⁴ cells/cm² was poured into wells of a 24-well plate to start culturing. After that, the culture medium in each well was exchanged every other day and cultured for approximately one month, and the following analysis was carried out.
(4) Observation was carried out over time using a phase contrast microscope and a fluorescence microscope to perform analysis of morphological changes of the multicellular population and determination of survival/death using calcein-AM and ethidium homodimer-1. FIG. 11A shows phase contrast microscope images and fluorescence microscope images of the control and the hepatic tissue-type chip on day 4 of culturing. In addition, FIG. 11B shows phase contrast microscope images and fluorescence microscope images of control and the hepatic tissue-type chip on day 32 of culturing.

From FIG. 11A, on day 4 of culturing, the control cells proliferated to form many non-uniform aggregates, and dead cells accumulated in the center portion of the aggregates. On the other hand, the cells of the hepatic tissue-type chip uniformly proliferated and formed a bile canaliculus-like structure and almost all survived.

In addition, from FIG. 11B, on day 32 of culturing, huge aggregates formed in the control cells and many dead cells were dispersed inside the aggregates. On the other hand, it was clear that the cells of the hepatic tissue-type chip formed a regular dense structure and almost all survived.

### [Example 4] Metabolism of Model Drug by HepG2 Cell Hepatic tissue-type chip and Excretion and Accumulation of Metabolites to Bile Canaliculus-like Structure 2

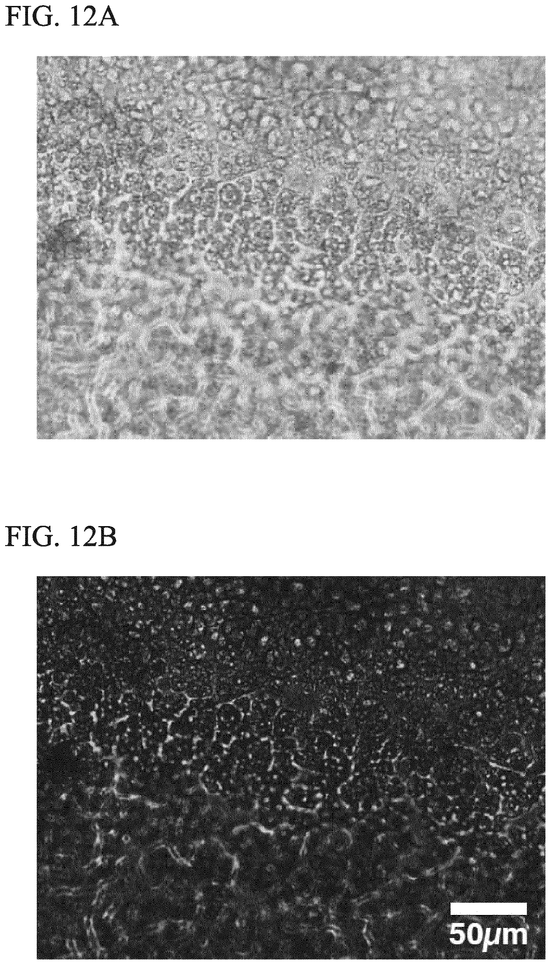

(1) HBSS containing fluorescein diacetate (FD) as a model drug at a concentration of 25 µg/mL was prepared, and 5 mL thereof was poured into a dish (60 mm in diameter).
(2) Next, on day 32 of culturing, the hepatic tissue-type chip produced using HepG2 cells in Example 3 was immersed in the FD-containing HBSS prepared in (1) and cultured for 1 hour such that the FD was taken into the cells.
(3) Next, the result was transferred into a new dish (diameter 60 mm) into which 5 mL of HBSS was poured, and washed. After performing this operation twice, it was confirmed by observation with a fluorescence microscope that green fluorescence (excitation wavelength: 490 nm, fluorescence wavelength: 514 nm) of the fluorescein metabolized by HepG2 cells was uniformly distributed in the cytoplasm.
(4) Next, the hepatic tissue-type chip was transferred into a new dish (diameter 60 mm) into which 5 mL of fresh HBSS was poured, immersed in HBSS, and cultured for 1 hour. The discharge of fluorescein in the cells after 1 hour was confirmed by observation with a phase contrast microscope and a fluorescence microscope (refer to FIG. 12A (phase contrast microscope image) and FIG. 12B (fluorescence microscope image)).

From FIG. 12A and FIG. 12B, it was confirmed that fluorescein distributed in the cytoplasm was excreted and accumulated in the bile canaliculus-like structure.

### [Example 5] Evaluation of Hepatic Tissue Specific Function in HepG2 Cell Hepatic Tissue-Type Chip 1

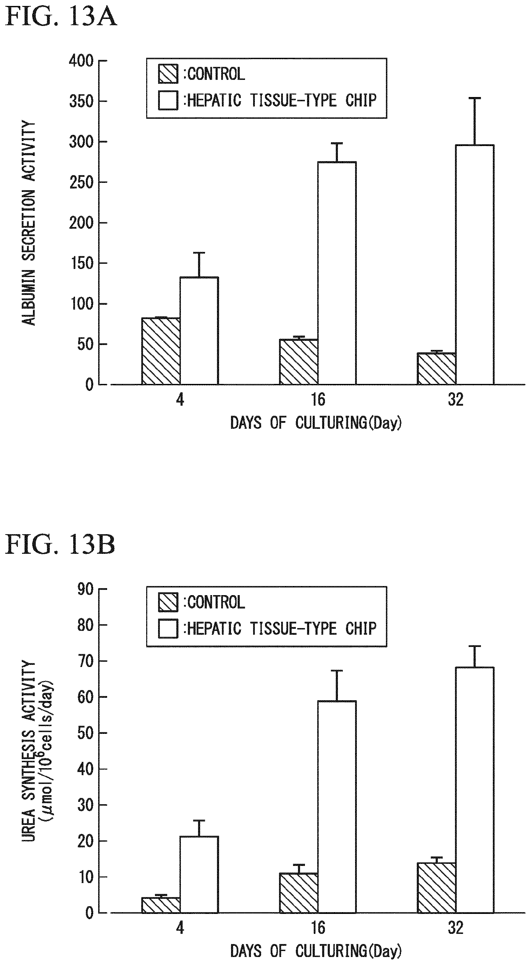

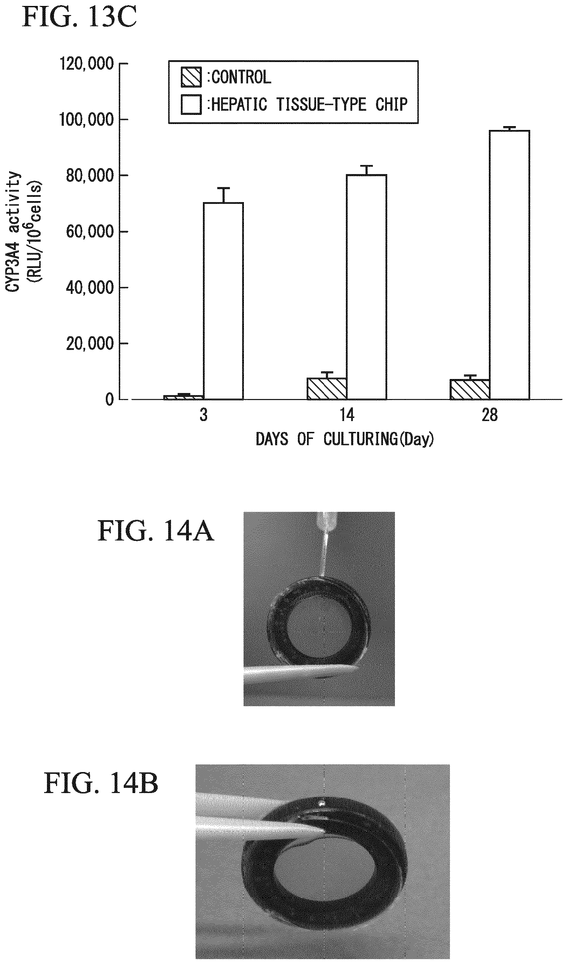

(1) In order to evaluate the hepatic tissue-specific function, for a hepatic tissue-type chip produced using HepG2 cells in Example 3, albumin synthesis (evaluation on days 4, 16, and 32 of culturing), urea synthesis (evaluation on days 4, 16, and 32 of culturing), and CYP3A4 activity (using day 3, 14, and 28 of culturing) were analyzed over time. Specifically, the albumin synthesis was measured using a Human Albumin ELISA Quantitation Set (manufactured by Bethyl Laboratories, Inc.) as a measurement kit. The urea synthesis was measured using QuantiChrom Urea Assay Kit (manufactured by BioAssay Systems) as a measurement kit. The CYP3A4 activity was measured using P450-Glo (registered trademark) CYP3A4 assay with Luciferin-IPA (manufactured by Promega) as a measurement kit. The specific activity was calculated from the number of seeded cells. In addition, in the same manner as for the control of Example 3, albumin synthesis (evaluation on days 4, 16, and 32 of culturing), urea synthesis (evaluation on days 4, 16, and 32 of culturing), and CYP3A4 activity (using day 3, 14, and 28 of culturing) were analyzed over time. The results are shown in FIG. 13A (albumin synthesis), FIG. 13B (urea synthesis), and FIG. 13C (CYP3A4 activity).

From FIG. 13A, FIG. 13B, and FIG. 13C, it is clear that, as compared with the control, the hepatic tissue-type chip was improved and that the improvement was maintained for approximately one month from the early stage of culturing in all of the albumin synthesis, the urea synthesis, and the CYP3A4 activity.

For CYP3A4 activity, differentiated HepaRG cells considered to be comparable to the average activity of frozen human primary hepatocytes were also compared and analyzed at the same time, and the CYP3A4 activity of the differentiated HepaRG cells was 207, 655±31, 111 (RLU/10⁶ cells). Accordingly, it was found that the CYP3A4 activity of the hepatic tissue-type chip reached a level of approximately 1/3 or more of the differentiated HepaRG cells.

### [Production Example 4] Production of Cell Enclosure Device (Using Cutting Processed and Molding Processed Plastic Ring) 4

(1) Large rings and small rings formed of plastic with wall holes were prepared. The large ring was provided with a wall hole (diameter 0.70 mm) with a thickness of 2.0 mm, an inner space volume of 1.0 mL, an inner diameter of 25.24 mm, and an inner bottom area of 5.00 cm². On the other hand, the small ring was provided with a wall hole (diameter 0.70 mm) with a thickness of 2.0 mm, an inner space volume of 0.1 mL, an inner diameter of 7.98 mm, and an inner bottom area of 0.50 cm².
   The wall holes of both large rings and small rings were plugged with stainless steel balls with a diameter of 800 µm. A collagen Vitrigel (registered trademark) membrane dried body prepared using the same method as in (1) of Production Example 1 was directly adhered to both surfaces of the large plastic ring and small ring using a polyurethane adhesive to produce a cell enclosure device.
(2) Next, with respect to the obtained cell enclosure device, it was confirmed that it is possible to enclose cells when a suspension of human dermal fibroblasts was safely injected from a wall hole using an indwelling needle catheter, and the wall hole was sealed with a stainless-steel ball (refer to FIG. 14A and FIG. 14B).

### [Example 6] Production of Dermal Tissue-Type Chip Using Human Dermal Fibroblasts 1

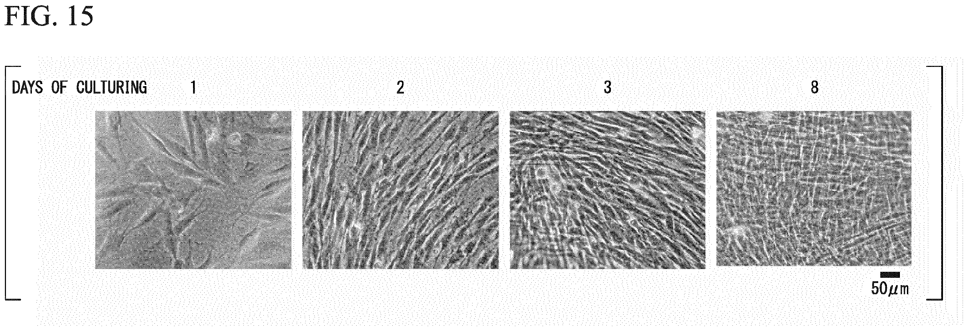

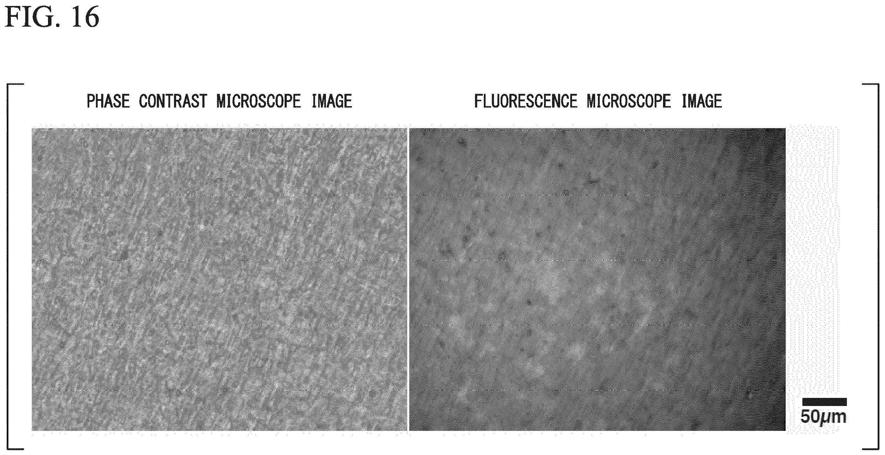

(1) Pre-cultured human dermal fibroblasts were collected and mixed with the culture medium so as to be 1.0 × 10⁵ cells/mL to prepare a suspension of human dermal fibroblasts.
(2) 100 µL of a suspension of human dermal fibroblasts (1.0 × 10⁵ cells/mL) were injected in a cell enclosure device produced using a plastic small ring in Production Example 4 from a wall hole using an indwelling needle catheter. Next, a dermal tissue-type chip was produced by plugging the wall hole with a stainless-steel ball. The initial seeding density was 2.0 × 10⁴/cm².
(3) 1.0 mL of the culture medium was poured into the wells of a 24-well plate, and the dermal tissue-type chip produced in (2) was immersed in the culture medium and the culturing was started. After that, the culture medium in each well was exchanged every other day and the cells were cultured for 21 days.
(4) Morphological changes of the multicellular population were analyzed by observation over time with a phase contrast microscope. FIG. 15 shows phase contrast microscope images of the dermal tissue-type chip on the days 1, 2, 3, and 8 of culturing.
   From FIG. 15, it was confirmed that human dermal fibroblasts proliferated satisfactorily and formed multilayers after adhering to a collagen Vitrigel (registered trademark) membrane.
(5) Furthermore, with respect to the dermal tissue-type chip, the culturing was continued, the culture medium in each well was exchanged every other day, and culturing was carried out until day 100 from the start of the culture.
(6) For the dermal tissue-type chip cultured for 100 days, phase contrast microscope observation and survival/death determination using calcein-AM and ethidium homodimer-1 were carried out. FIG. 16 shows a phase contrast microscope image and a fluorescence microscope image of a dermal tissue-type chip cultured for 100 days.

From FIG. 16, it was clear that the cells of the dermal tissue-type chip cultured for 100 days proliferated and formed multilayers with a regular dense structure, and most of the cells survived.

### [Example 7] Collection of Human Dermal Fibroblasts from a Dermal Tissue-Type Chip 1

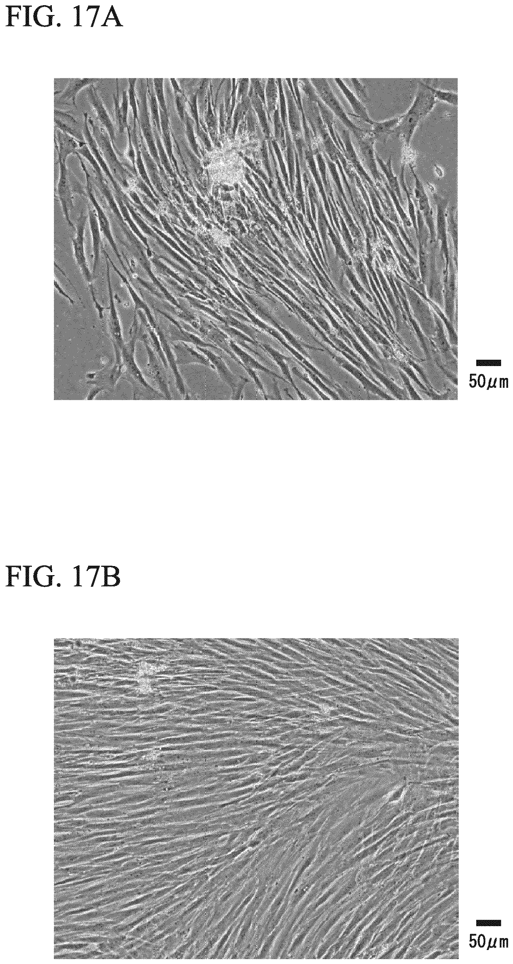

(1) Whether it was possible to collect human dermal fibroblasts from dermal tissue-type chips was examined by treating the dermal tissue-type chip produced in Example 6 on day 15 of culturing with 0.5 mg/mL collagenase. As a result, it was clear that, by treating at 37°C for 20 minutes, the collagen Vitrigel (registered trademark) membrane was digested and it was possible to separate human dermal fibroblasts from the device.
(2) Next, the collected human dermal fibroblasts were cultured in a plastic culture dish and observed over time using a phase contrast microscope. Phase contrast microscope images of human dermal fibroblasts on day 2 and day 7 of culture are shown in FIG. 17A (day 2 of culturing) and FIG. 17B (day 7 of culturing).

From FIG. 17A and FIG. 17B, it was clear that the human dermal fibroblasts collected from the dermal tissue-type chip proliferated satisfactorily.

### [Example 8] Collection of Human Dermal Fibroblasts from a Dermal Tissue-Type Chip 2

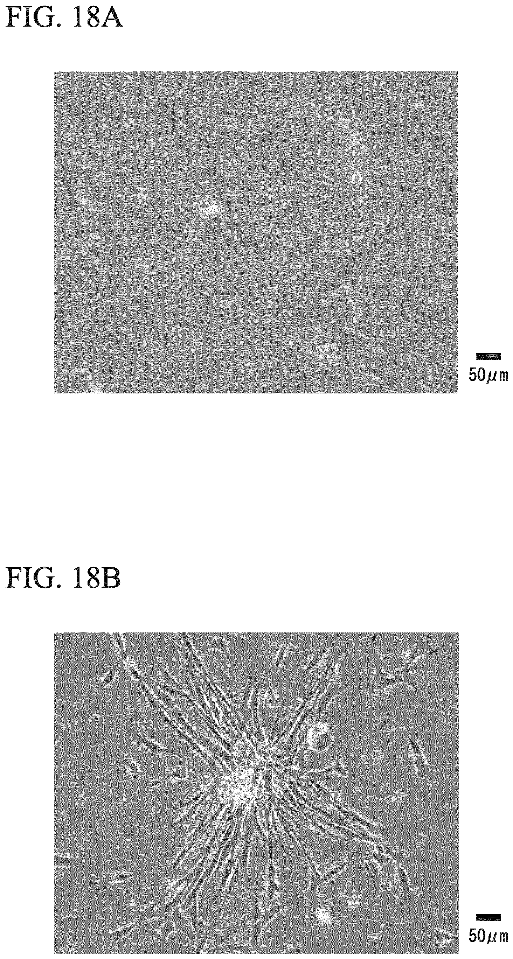

(1) A collagen Vitrigel (registered trademark) membrane was cut out along the inner edge of a plastic ring with ophthalmic scissors on day 21 of the culturing of the dermal tissue-type chip produced in Example 6. Next, the membrane was transferred to a plastic culture dish in which a culture medium was poured, and many small cut pieces were obtained by cutting into small pieces. Next, in order to examine whether it is possible to separate human dermal fibroblasts by culturing this cut piece, observation was carried out over time using a phase contrast microscope. Phase contrast microscope images of human dermal fibroblasts 30 minutes after the start of culturing and on day 1 of culturing are shown in FIG. 18A (30 minutes after the start of culturing) and FIG. 18B (day 1 of culturing).

From FIG. 18A and FIG. 18B, it was clear that the human dermal fibroblasts collected from the dermal tissue-type chip proliferated satisfactorily.

### [Example 9] Production of Hepatic Tissue-Type Chip Using HepG2 Cell of Human Liver Cancer Cell Line 3

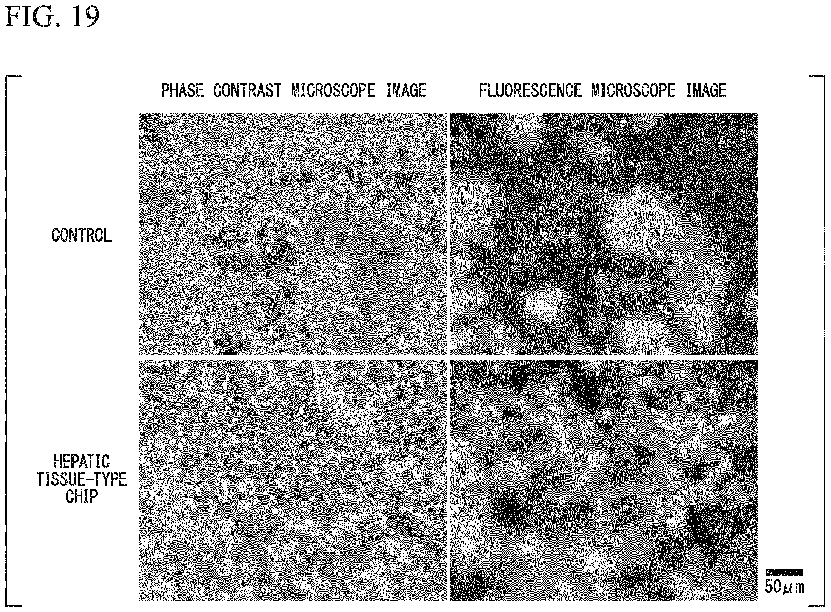

(1) Pre-cultured HepG2 cells (purchased from RIKEN BioResource Center, RCB 1648) were collected and mixed with a culture medium so as to be 2.5 × 10⁵ cells/mL to prepare a suspension of HepG2 cells.
(2) In a cell enclosure device produced using a plastic small ring in Production Example 4, 100 µL of a suspension (2.5 × 10⁵ cells/mL) of HepG2 cells was injected from a wall hole using an indwelling needle catheter. Next, the wall hole was plugged with a stainless-steel ball to produce a hepatic tissue-type chip. The initial seeding density was 5.0 × 10⁴/cm².
(3) 1.0 mL of the culture medium was poured into wells of a 24-well plate, the hepatic tissue-type chip produced in (2) was immersed in the culture medium and the culturing was started. As a control, 1.0 mL of a suspension of HepG2 cells prepared to have an initial seeding density of 5.0 × 10⁴ cells/cm² was poured into wells of a 24-well plate to start culturing. After that, the culture medium in each well was exchanged every other day and cultured for approximately one month, and the following analysis was carried out.
(4) Observation was carried out over time using a phase contrast microscope and a fluorescence microscope to perform the analysis of morphological changes of multicellular population and determination of survival/death using calcein-AM and ethidium homodimer-1. FIG. 19 shows phase contrast microscope images and fluorescence microscope images of control and hepatic tissue-type chips on day 28 of culturing.

From FIG. 19, on day 28 of culturing, huge aggregates formed in the control cells and many dead cells were dispersed inside the aggregates. On the other hand, it was clear that the cells of the hepatic tissue-type chip formed a regular dense structure and almost all survived.

### [Example 10] Metabolism of Model Drug by HepG2 Cell Hepatic Tissue-Type Chip and Excretion and Accumulation of Metabolites to Bile Canaliculus-Like Structure 3

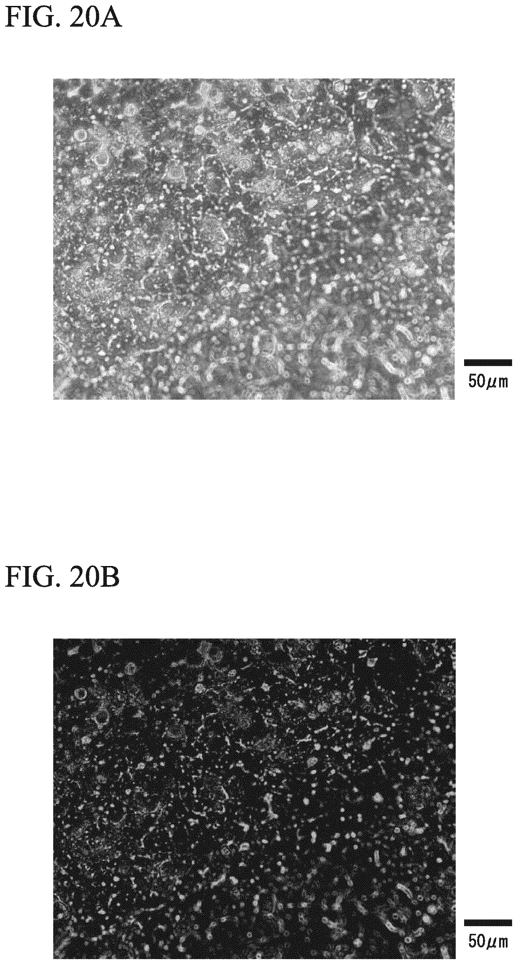

(1) HBSS containing fluorescein diacetate (FD) as a model drug at a concentration of 25 µg/mL was prepared, and 5 mL thereof was poured into a dish (diameter 60 mm).
(2) Next, the hepatic tissue-type chip on day 35 of culturing produced using HepG2 cells in Example 9 was immersed in the FD-containing HBSS prepared in (1) and cultured for 1 hour such that the FD was taken into the cells.
(3) Next, the result was transferred into a new dish (diameter 60 mm) into which 5 mL of HBSS was poured, and washed. After performing this operation twice, it was confirmed by observation with a fluorescence microscope that green fluorescence (excitation wavelength: 490 nm, fluorescence wavelength: 514 nm) of the fluorescein metabolized by HepG2 cells was uniformly distributed in the cytoplasm.
(4) Next, the hepatic tissue-type chip was transferred into a new dish (diameter 60 mm) into which 5 mL of fresh HBSS was poured, immersed in HBSS, and cultured for 1 hour. The discharge of fluorescein in the cells after 1 hour was confirmed by observation with a phase contrast microscope and a fluorescence microscope (refer to FIG. 20A (phase contrast microscope image) and FIG. 20B (fluorescence microscope image)).

From FIG. 20A and FIG. 20B, it was confirmed that fluorescein distributed in the cytoplasm was excreted and accumulated in the bile canaliculus-like structure.

### [Example 11] Evaluation of Hepatic Tissue Specific Function in Hepatic Tissue-Type Chips of HepG2 Cells 2

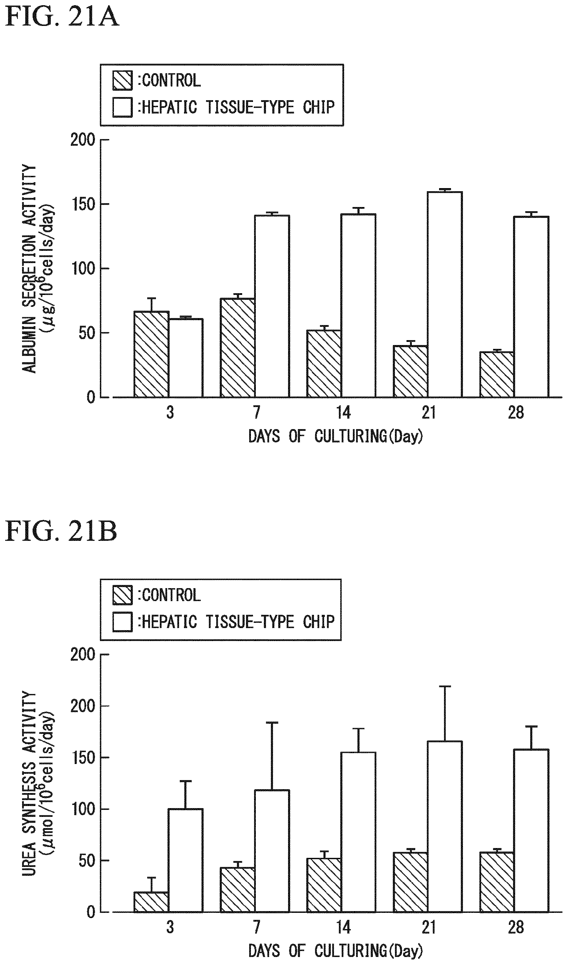

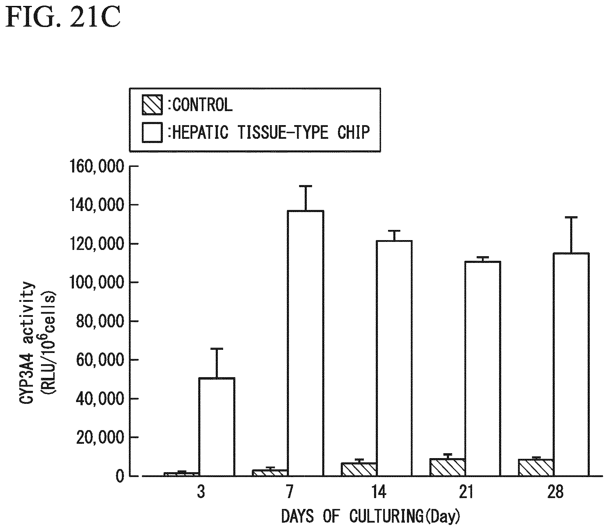

(1) In order to evaluate the hepatic tissue specific function, albumin synthesis, urea synthesis, and CYP3A4 activity were analyzed over time for hepatic tissue-type chips produced using HepG2 cells in Example 9 (evaluated on days 3, 7, 14, 21, and 28 of culturing). Specifically, the albumin synthesis was measured using a Human Albumin ELISA Quantitation Set (manufactured by Bethyl Laboratories, Inc.) as a measurement kit. The urea synthesis was measured using QuantiChrom Urea Assay Kit (manufactured by BioAssay Systems) as a measurement kit. The CYP3A4 activity was measured using P450-Glo (registered trademark) CYP3A4 assay with Luciferin-IPA (manufactured by Promega) as a measurement kit. The specific activity was calculated from the number of seeded cells. In addition, for the control of Example 9, the albumin synthesis, urea synthesis, and CYP3A4 activity were analyzed in the same manner over time (evaluation on days 3, 7, 14, 21, and 28 of culturing). The results are shown in FIG. 21A (albumin synthesis), FIG. 21B (urea synthesis), and FIG. 21C (CYP3A4 activity).

From FIG. 21A, FIG. 21B, and FIG. 21C, it is clear that, as compared with the control, the hepatic tissue-type chip was improved and the improvement was maintained for approximately one month from the early stage of culturing in all of the albumin synthesis, the urea synthesis, and the CYP3A4 activity.

For CYP3A4 activity, differentiated HepaRG cells considered to be comparable to the average activity of frozen human primary hepatocytes were also compared and analyzed at the same time, and the CYP3A4 activity of the differentiated HepaRG cells was 179, 447±15, 287 (RLU/10⁶ cells). Accordingly, it was found that the CYP3A4 activity of hepatic tissue-type chips cultured for 7 days or more reached the level of approximately half or more of the differentiated HepaRG cells.

### [Production Example 5] Production of Cell Enclosure Device (Using Cutting Processed and Molding Processed Plastic Ring, Collagen Vitrigel (Registered Trademark) Membrane Dried Body on One Surface, Dialysis Membrane Adhered to Other Side) 5

(1) A plastic small ring with wall hole was prepared. The size of the small ring was 7.98 mm in inner diameter, 13.0 mm in outer diameter, and 2.0 mm in thickness (inner space volume 0.1 mL, inner bottom area 0.50 cm²). A collagen Vitrigel (registered trademark) membrane dried body prepared using the same method as in (1) of Production Example 1 was directly adhered to one side of the plastic small ring using a polyurethane adhesive.
(2) Next, a dialysis membrane (cellulose tube for dialysis membrane, cut-off of molecular weight: 14,000, manufactured by Sanko Junyaku Co., Ltd.) was prepared, hydrated, and the tube was cut open. Next, in a state where the cut open dialysis membrane was sandwiched between the ring-shaped magnets together with the support film and dried, the dialysis membrane was directly adhered to the other side of the small ring using a polyurethane adhesive. Next, the support film was removed, and an extra portion of the outer periphery of the ring was cut to produce a cell enclosure device.

### [Production Example 6] Production of Cell Enclosure Device (Using Cutting Processed and Molding Processed Plastic Ring, Collagen Vitrigel (Registered Trademark) Membrane Dried Body on One Surface, Membrane Filter Adhered to Other Side) 6

(1) A plastic small ring with wall hole was prepared. The size of the small ring was 7.98 mm in inner diameter, 13.0 mm in outer diameter, and 2.0 mm in thickness (inner space volume 0.1 mL, and inner bottom area 0.50 cm²). A collagen Vitrigel (registered trademark) membrane dried body prepared using the same method as in (1) of Production Example 1 was directly adhered to one side of the plastic small ring using a polyurethane adhesive.
(2) Next, a PTFE membrane filter (Omnipore (registered trademark) membrane, diameter: 13.0 mm, pore size: 0.2 µm and 0.45 µm, manufactured by Millipore) was prepared, wetted with 70% ethanol, sterilized, and dried. Next, each of the dried membrane filters was directly adhered to the other surface of the small ring using a polyurethane adhesive to produce two types of cell enclosure devices having different membrane pore sizes.

### [Example 12] Protein Permeability Test for Cell Enclosure Device 1

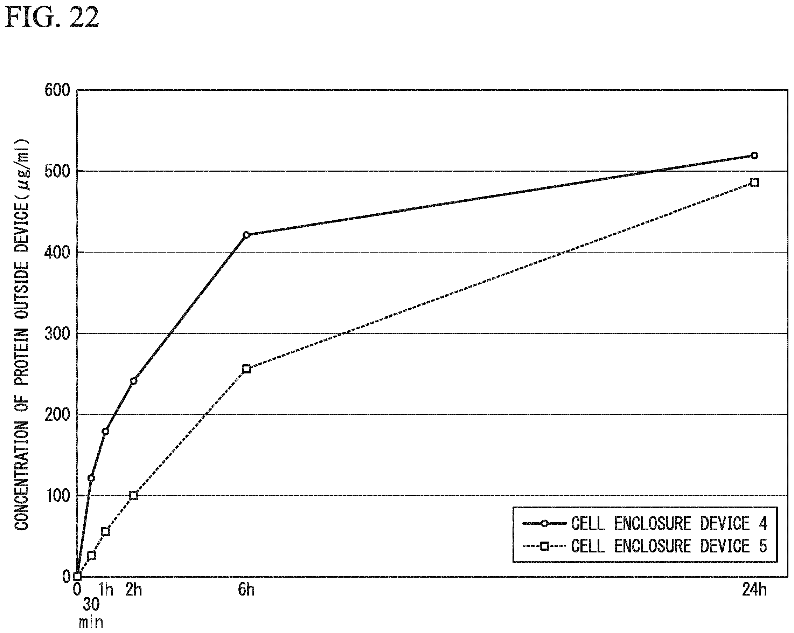

(1) A cell enclosure device produced using a plastic small ring with a wall hole in Production Example 4 in which a collagen Vitrigel (registered trademark) membrane dried body was attached on both surfaces (may be referred to below as "cell enclosure device 4") and a cell enclosure device produced using a plastic small ring with a wall hole in Production Example 5 in which a collagen Vitrigel (registered trademark) membrane dried body was attached on one surface and a dialysis membrane was attached on the other surface (may be referred to below as "cell enclosure device 5") were prepared.
(2) Next, 100 µL of a 30% FBS solution was injected into the cell enclosure device 4 and the cell enclosure device 5. The 30% FBS solution was prepared by mixing 3.0 mL of FBS and 7.0 mL of PBS.
(3) Next, 3.0 mL of PBS was dispensed into each well of a 12-well plate.
(4) Subsequently, the cell enclosure device 4 and the cell enclosure device 5 enclosing 100 µL of the 30% FBS solution were submerged one by one in each well of a 12-well plate such that the lower surfaces thereof were the collagen Vitrigel (registered trademark) membranes. Next, shaking (70 rpm) was started in a 37°C incubator.
(5) Next, the amount of protein permeated from the inside of the device to the outside of the device over time was measured (30 minutes, 1 hour, 2 hours, 6 hours, and 24 hours after the start of shaking). The results are shown in FIG. 22.

From FIG. 22, as a result of comparing the protein permeability of the cell enclosure device 4 and the cell enclosure device 5, it was found that the protein permeability of the cell enclosure device 5 was inferior to the cell enclosure device 4.

### [Example 13] Protein Permeability Test for Cell Enclosure Device 2

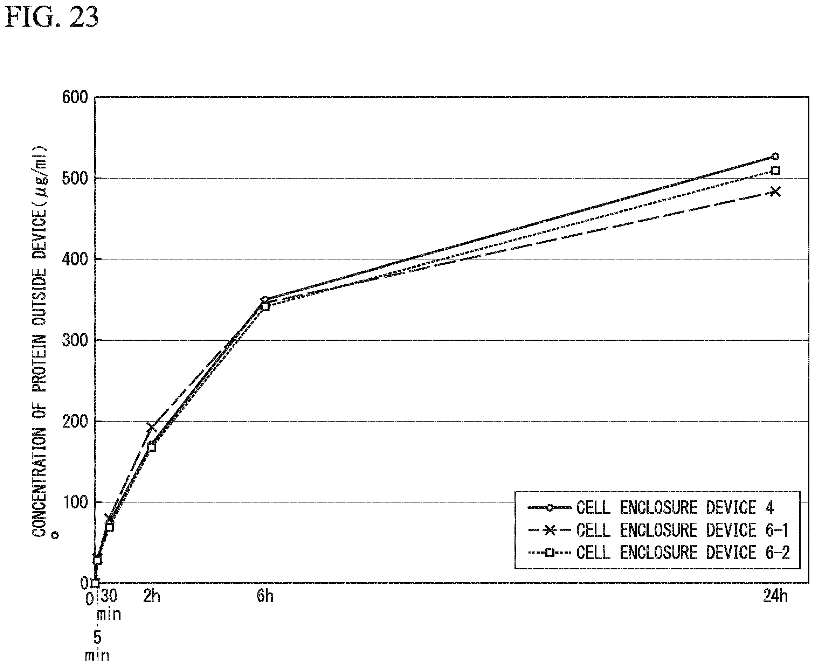

(1) A cell enclosure device produced using a plastic small ring with a wall hole in Production Example 4 in which a collagen Vitrigel (registered trademark) membrane dried body is attached on both sides (may be referred to below as "cell enclosure device 4"), a cell enclosure device produced using a plastic small ring with a wall hole in Production Example 6 in which a collagen Vitrigel (registered trademark) membrane dried body is attached to one side and a PTFE membrane filter (pore size: 0.2 µm) is attached to the other side (may be referred to below as "cell enclosure device 6-1"), and a cell enclosure device produced using a plastic small ring with a wall hole in Production Example 6 in which a collagen Vitrigel (registered trademark) membrane dried body is attached on one side and a PTFE membrane filter (pore size: 0.45 µm) is attached to the other side (may be referred to below as "cell enclosure device 6-2"), were prepared.
(2) Next, 100 µL of a 30% FBS solution was injected into the cell enclosure device 4, the cell enclosure device 6-1, and the cell enclosure device 6-2. The 30% FBS solution was prepared by mixing 3.0 mL of FBS and 7.0 mL of PBS.
(3) Next, 3.0 mL of PBS was dispensed into each well of a 12-well plate.
(4) Next, the cell enclosure device 4, the cell enclosure device 6-1, and the cell enclosure device 6-2 in which 100 µL of a 30% FBS solution was enclosed were immersed in each well of a 12-well plate one by one such that the lower surfaces thereof were the collagen Vitrigel (registered trademark) membrane. Next, shaking (70 rpm) was started in a 37°C incubator.
(5) Next, the amount of protein permeated from the inside of the device to the outside of the device over time was measured (5 minutes, 30 minutes, 2 hours, 6 hours, and 24 hours after the start of shaking). The results are shown in FIG. 23.

From FIG. 23, as a result of comparing the protein permeability of the cell enclosure device 4, the cell enclosure device 6-1, and the cell enclosure device 6-2, it was found that there was no significant difference between the cell enclosure devices.

### [Example 14] Production of Hepatic Tissue-Type Chip Using HepG2 Cell of Human Liver Cancer Cell Line 4 and Storage Test 1

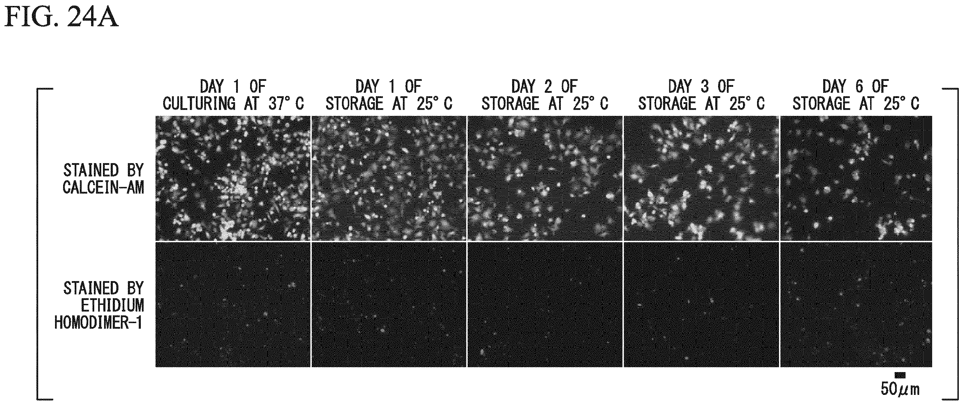

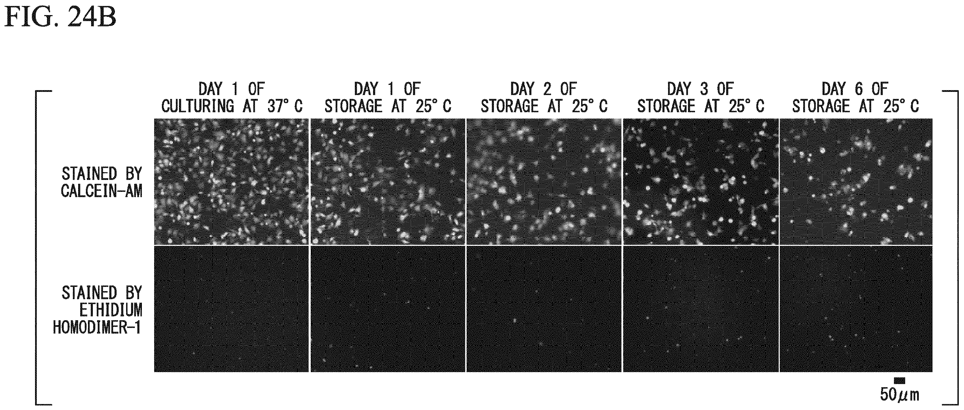

(1) Pre-cultured HepG2 cells (purchased from RIKEN BioResource Center, RCB 1648) were collected and mixed with a culture medium so as to be 2.5 × 10⁵ cells/mL to prepare a suspension of HepG2 cells.
(2) A suspension (2.5 × 10⁵ cells/mL) of 100 µL of HepG2 cells was injected into the cell enclosure device 4 produced in Production Example 4 from a wall hole using an indwelling needle catheter. Next, the wall hole was plugged with a stainless-steel ball to produce a hepatic tissue-type chip (may be referred to below as "hepatic tissue-type chip 4"). The initial seeding density was 5.0 × 10⁴/cm². Five hepatic tissue-type chips were produced.
(3) In addition, a suspension (2.5 × 10⁵ cells/mL) of 100 µL of HepG2 cells was injected into the cell enclosure device 6-1 produced in Production Example 6 from the wall hole using an indwelling needle catheter. Next, the wall hole was plugged with a stainless-steel ball to produce a hepatic tissue-type chip (referred to below as "hepatic tissue-type chip 6-1"). The initial seeding density was 5.0 × 10⁴/cm². Five hepatic tissue-type chips were prepared.
(4) Next, 1.0 mL of the culture medium was poured into wells of a 24-well plate, and the hepatic tissue-type chip 4 and the hepatic tissue-type chip 6-1 produced in (2) and (3) were immersed in a culture medium to start culturing in a 37°C humidified incubator in the presence of 5% CO₂.
(5) After culturing for 24 hours (a whole day), survival/death determination was carried out using calcein-AM and ethidium homodimer-1 one by one for each hepatic tissue-type chip. As a result, in all the devices, it was confirmed that the cells adhered satisfactorily to the collagen Vitrigel (registered trademark) membrane and survived (refer to "Day 1 of culturing at 37°C" of FIG. 24A ("hepatic tissue-type chip 4") and FIG. 24B ("Hepatic tissue-type chip 6-1")).
(6) Next, for each of the remaining four hepatic tissue-type chips, a 15 mL conical tube into which the culture medium was poured was prepared and each hepatic tissue-type chip was transferred one by one into the conical tube. Next, the conical tube was filled with a culture medium so as to further prevent gas from entering, and the tube was sealed.
(7) Next, a conical tube in which the hepatic tissue-type chip 4 or the hepatic tissue-type chip 6-1 were sealed one by one were transferred into a 25°C gas phase incubator, and storage at 25°C was started.
(8) Each hepatic tissue-type chip stored at 25°C was extracted one by one on days 1, 2, 3, and 6 after storage. Immediately after that, the survival/death determination was carried out using calcein-AM and ethidium homodimer-1. The results are shown in FIG. 24A ("hepatic tissue-type chip 4") and FIG. 24B ("hepatic tissue-type chip 6-1").

From FIG. 24A and FIG. 24B, in storage at 25°C for 3 days or more, calcein-positive cells decreased in both the hepatic tissue-type chip 4 and the hepatic tissue-type chip 6-1 compared to before storage. On the other hand, it was found that, in any storage period at 25°C, the ethidium homodimer-1 positive cells were equally small in both the hepatic tissue-type chip 4 and the hepatic tissue-type chip 6-1, compared with before storage. The above suggests that it is possible to satisfactorily maintain the viable cells for 6 days in both the hepatic tissue-type chip 4 and the hepatic tissue-type chip 6-1 immediately after storage at 25°C.

### [Example 15] Production of Hepatic Tissue-Type Chip Using HepG2 Cell of Human Liver Cancer Cell Line 5 and Storage Test 2

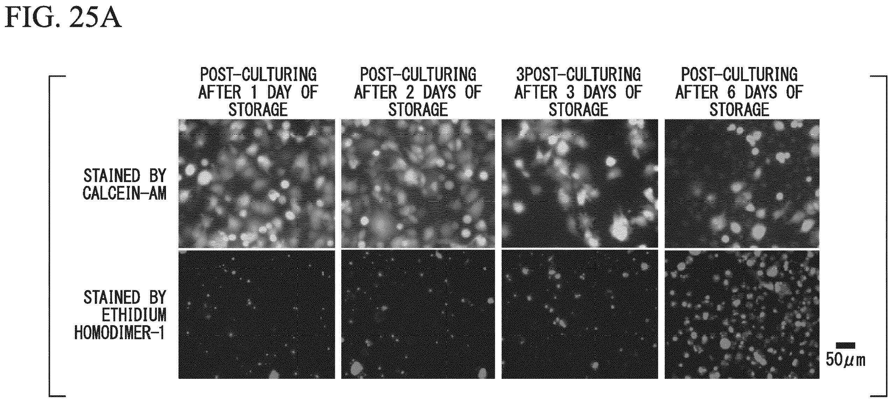

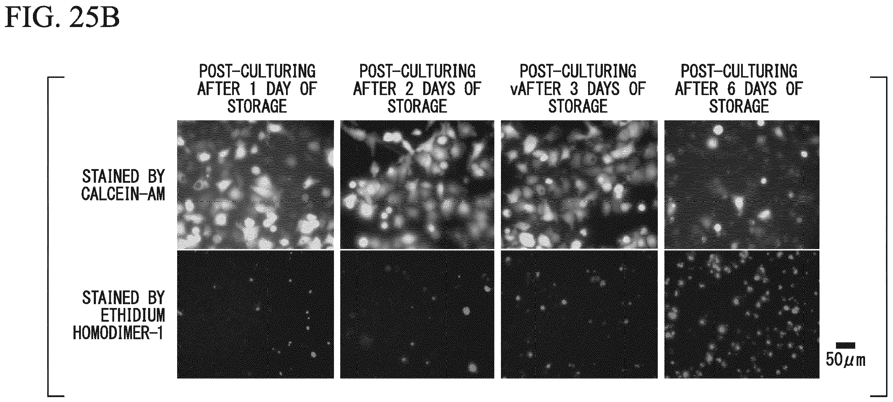

(1) Pre-cultured HepG2 cells (purchased from RIKEN BioResource Center, RCB 1648) were collected and mixed with the culture medium so as to be 2.5 × 10⁵ cells/mL to prepare a suspension of HepG2 cells.
(2) A suspension (2.5 × 10 5 cells/mL) of 100 µL of HepG2 cells was injected into the cell enclosure device 4 produced in Production Example 4 from a wall hole using an indwelling needle catheter. Next, the wall hole was plugged with a stainless-steel ball to produce a hepatic tissue-type chip (referred to below as "hepatic tissue-type chip 4"). The initial seeding density was 5.0 × 10⁴/cm². Four hepatic tissue-type chips were prepared.
(3) In addition, a suspension (2.5 × 10⁵ cells/mL) of 100 µL of HepG2 cells was injected into the cell enclosure device 6-1 produced in Production Example 6 from the wall hole using an indwelling needle catheter. Next, the wall hole was plugged with a stainless-steel ball to produce a hepatic tissue-type chip (referred to below as "hepatic tissue-type chip 6-1"). The initial seeding density was 5.0 × 10⁴/cm². Four hepatic tissue-type chips were produced.
(4) Next, 1.0 mL of a culture medium was poured into wells of a 24-well plate, and the hepatic tissue-type chip 4 and the hepatic tissue-type chip 6-1 produced in (2) and (3) were immersed in a culture medium and culturing was started in a 37°C humidified incubator in the presence of 5% CO₂.
(5) After culturing for 24 hours (a whole day), a 15 mL conical tube into which the culture medium was poured was prepared, and each hepatic tissue-type chip was transferred one by one into the conical tube. Next, the conical tube was filled with a culture medium so as to further prevent gas from entering, and the tube was sealed.
(6) Next, conical tubes in which the hepatic tissue-type chip 4 or the hepatic tissue-type chip 6-1 was sealed one by one were transferred into a 25°C gas phase incubator, and storage at 25°C was started.
(7) Each hepatic tissue-type chip stored at 25°C was extracted one by one on days 1, 2, 3, and 6 after storage. Next, 1.0 mL of the culture medium was poured into wells of a 24-well plate, each of the extracted hepatic tissue-type chips was immersed in the culture medium and post-cultured in a 37°C humidified incubator in the presence of 5% CO² for 24 hours (a whole day). After post-culturing for 24 hours (one whole day) at 37°C, survival/death judgment was carried out using calcein-AM and ethidium homodimer-1. The results are shown in FIG. 25A ("hepatic tissue-type chip 4") and FIG. 25B ("hepatic tissue-type chip 6-1").

From FIG. 25A and FIG. 25B, it was found that calcein-positive cells are decreased in the hepatic tissue-type chip 4 when stored at 25°C for 3 days or more, and in the hepatic tissue-type chip 6-1 when stored at 25°C for 6 days. On the other hand, it was found that ethidium homodimer-1 positive cells were increased in the hepatic tissue-type chip 4 when stored at 25°C for 3 days or more, and in the hepatic tissue-type chip 6-1 when stored at 25°C for 6 days.

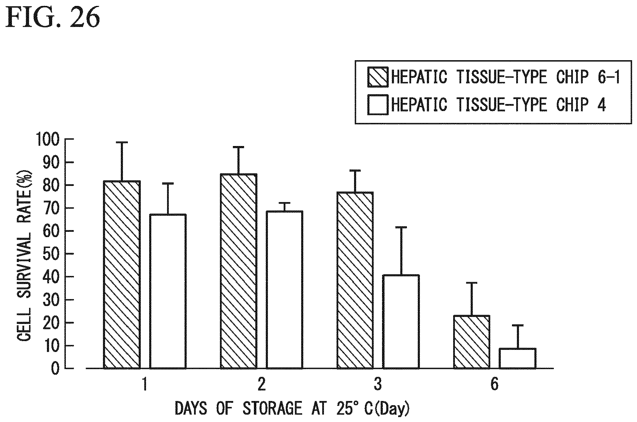

Therefore, using the fluorescence microscope images of FIG. 25A and FIG. 25B, three arbitrary places where squares with sides of 100 µm do not overlap were selected, positive cells of calcein and ethidium homodimer-1 in the squares were measured, and the cell survival rate was analyzed. The results are shown in FIG. 26.

From FIG. 26, in the hepatic tissue-type chip 6-1, the cell survival rate was maintained more satisfactorily than in the hepatic tissue-type chip 4 even in post-culturing after storage at 25°C. In particular, it was found that, in the hepatic tissue-type chip 6-1, it is possible to maintain a cell survival rate of 77.3 ± 9.2% on day 1 of post-culturing at 37°C after storage at 25°C for 3 days.

### [Production Example 7] Production of Cell Enclosure Device (With an Indwelling Needle Catheter, Formed Only of Atelocollagen Vitrigel (registered trademark)) 7

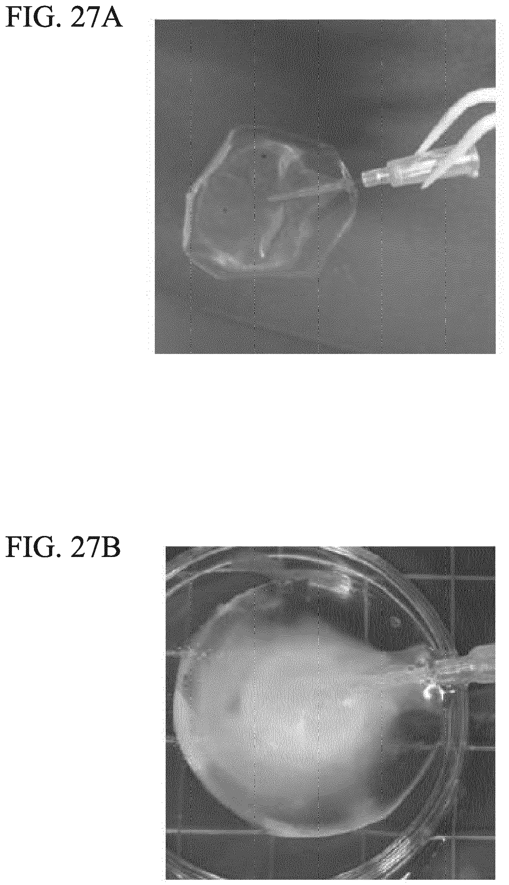

(1) An atelocollagen Vitrigel (registered trademark) membrane dried body (collagen amount: 5.5 mg/cm²) was produced by pouring 10.0 mL of 0.5% atelocollagen sol into a walled mold having an inner diameter of 34 mm according to a known method (reference: PCT International Publication No. WO 2012/026531).
   The 0.5% atelocollagen sol was prepared by dispensing 6 mL of a serum-free culture medium on ice into 50 mL conical tubes, then adding 6 mL of a porcine-derived atelocollagen solution (manufactured by Kanto Chemical Co., Inc., collagen concentration 1.0 mass%), and performing pipetting three times. In addition, the serum-free culture medium used had the following composition.
   Serum-free culture medium: Dulbecco's Modified Eagle's Medium (DMEM) (Cat. No. 11885-084, manufactured by GIBCO)
   +20 mM HEPES (manufactured by GIBCO, Cat. No. 15630-080)
   +100 units/mL penicillin + 100 µg/mL streptomycin (manufactured by GIBCO, Cat. No. 15140-148)
(2) Next, two of the atelocollagen Vitrigel (registered trademark) membrane dried bodies produced in (1) were rehydrated with PBS to prepare two atelocollagen Vitrigel (registered trademark) membranes. Next, one atelocollagen Vitrigel (registered trademark) membrane was then stretched on a vinyl sheet and 0.5 mL of 0.5% atelocollagen sol was added thereon to spread out without protruding. An indwelling needle catheter was placed such that the tip of the indwelling needle catheter ("Nipro Safelet Cath NIC * 26 × 3/4 (trade name)" manufactured by Nipro, catheter, gauge number: 26G, outer diameter: 0.6 mm, and length: 19 mm) approached the approximate center of the atelocollagen Vitrigel (registered trademark) membrane. Next, the result was covered with one more atelocollagen Vitrigel (registered trademark) membrane.
   The 0.5% atelocollagen sol was used as an adhesive in accordance with a known method (reference: Japanese Unexamined Patent Application, First Publication No. 2015-203018).
(3) Next, the result was dried (vitrified) using a clean air dryer in an incubator at 10°C and 40% humidity.
(4) Next, a silicone ring (11.8 mm in inner diameter × 2.4 mm in thickness) was wrapped in aluminum foil and overlapped so as to be centered on the center of the circular atelocollagen Vitrigel (registered trademark) membrane dried bodies sandwiching the indwelling needle catheter, such that the inside of the atelocollagen Vitrigel (registered trademark) membrane dried body was masked. Next, UV irradiation (total UV irradiation amount per unit area: 400 mJ/cm²) was performed.
(5) The atelocollagen Vitrigel (registered trademark) membrane dried body after UV irradiation was inverted, the inside of the atelocollagen Vitrigel (registered trademark) membrane dried body was masked in the same manner, and UV irradiation (total UV irradiation amount per unit area: 400 mJ/cm²) was performed.
(6) Next, vitrification was allowed to proceed in a dish or the like to produce a cell enclosure device with an indwelling needle catheter (refer to FIG. 27A).
(7) Next, with respect to the obtained cell enclosure device, it was confirmed that it was possible to safely inject a suspension of human dermal fibroblasts from the indwelling needle catheter and enclose the cells (refer to FIG. 27B).

### [Example 16] Production of Dermal Tissue-Type Chip Using Human Dermal Fibroblasts 2

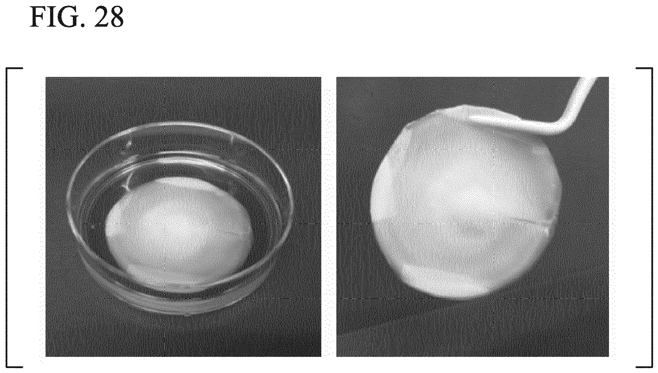

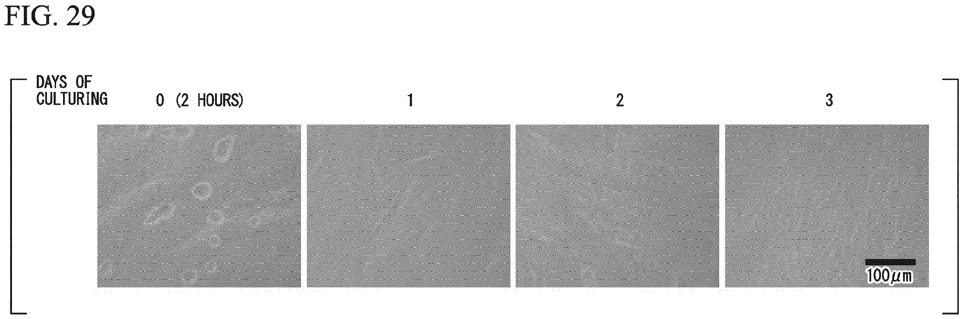

(1) Pre-cultured human dermal fibroblasts were collected and mixed with the culture medium so as to be 5.2 × 10⁵ cells/mL to prepare a suspension of human dermal fibroblasts.
(2) Next, the cell enclosure device with the indwelling needle catheter produced in Production Example 7 was rehydrated with a culture medium, then a syringe was connected to the indwelling needle catheter and 600 µL of a suspension of human dermal fibroblasts (5.2 × 10⁵ cells/mL) was injected to produce a dermal tissue-type chip. Next, the indwelling needle catheter was lightly pulled and removed from the dermal tissue-type chip. At this time, the suspension injected into the cell enclosure device leaked almost halfway to the outside, but it was possible to maintain a state in which approximately half were injected. Therefore, it was not possible to calculate the initial seeding density.
(3) Next, 5.0 mL of the culture medium was poured into a 6 cm diameter dish, and the dermal tissue-type chip produced in (2) was immersed in the culture medium and culturing was started. After that, the culture medium in each well was exchanged every other day and cultured for approximately three days. FIG. 28 shows an image photographing a dermal tissue-type chip on day 1 of culturing.
(4) Morphological changes of multicellular populations were analyzed by observation over time with a phase contrast microscope. FIG. 29 shows a phase contrast microscope image of the dermal tissue-type chip in culturing on days 0 (2 hours), 1, 2, and 3.

From FIG. 29, it was confirmed that human dermal fibroblasts adhered to the atelocollagen Vitrigel (registered trademark) membrane and proliferated satisfactorily.

### [Production Example 8] Production of Cell Enclosure Device (Formed Only of Atelocollagen Vitrigel (registered trademark)) 8

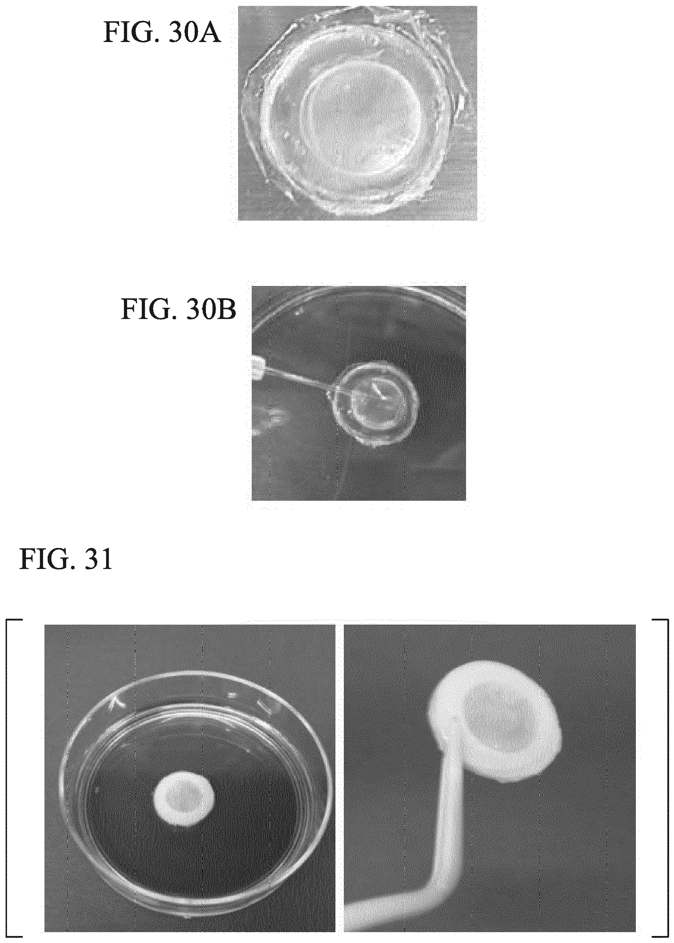

(1) Four sheets of atelocollagen Vitrigel (registered trademark) membrane dried bodies were produced using the same method as in (1) of Production Example 7. For each of the 4 atelocollagen Vitrigel (registered trademark) membrane dried bodies, each membrane thickness was measured with a Digimatic micrometer (manufactured by Mitutoyo Corporation), and the results were 74 ± 11 µm.
(2) Next, two atelocollagen Vitrigel (registered trademark) membrane dried bodies produced in (1) were rehydrated with PBS to prepare two atelocollagen Vitrigel (registered trademark) membranes. One atelocollagen Vitrigel (registered trademark) membrane was stretched on a vinyl sheet. Next, 0.5 mL of 0.5% atelocollagen sol was added thereon to spread out without protruding and then covered with another atelocollagen Vitrigel (registered trademark) membrane.
   The 0.5% atelocollagen sol was used as an adhesive in accordance with a known method (reference: Japanese Unexamined Patent Application, First Publication No. 2015-203018). For the remaining two atelocollagen Vitrigel (registered trademark) membranes, two sets of double layers of two atelocollagen Vitrigel (registered trademark) membrane dried bodies were produced by repeating the same operation as above.
(3) Next, the result was dried (vitrified) using a clean air dryer in an incubator at 10°C and 40% humidity.
(4) Next, UV irradiation (total UV irradiation amount per unit area: 400 mJ/cm²) was performed. Furthermore, UV irradiation (total UV irradiation amount per unit area: 400 mJ/cm²) was performed by inverting the atelocollagen Vitrigel (registered trademark) membrane double layer adhesive dried bodies after UV irradiation.
(5) Next, the double layer adhesive dried bodies of two atelocollagen Vitrigel (registered trademark) membrane dried bodies were rehydrated with PBS. Next, two atelocollagen Vitrigel (registered trademark) membrane double layer adherents with a diameter of 13 mm were then cut out from a pair of double layer adherents using a punch with a diameter of 13 mm and a total of four 13 mm diameter atelocollagen Vitrigel (registered trademark) membrane double layer adherents were prepared.
(6) Next, one 13 mm diameter atelocollagen Vitrigel (registered trademark) membrane double layer adherent was stretched on a vinyl sheet. Next, 90 µL of 0.5% atelocollagen sol was added on top and spread out so as to not protrude and then covered with another 13 mm diameter atelocollagen Vitrigel (registered trademark) membrane double layer adherent to prepare a quadruple layer body. Furthermore, 90 µL of 0.5% atelocollagen sol was added onto this quadruple layer body to spread out so as to not protrude, and then another 13 mm diameter atelocollagen Vitrigel (registered trademark) membrane double layer adherent was overlaid thereon to produce a six-layer body. Furthermore, 90 µL of 0.5% atelocollagen sol was added onto the six-layer body so as to not protrude and then covered with another 13 mm diameter atelocollagen Vitrigel (registered trademark) membrane double layer adherent to produce an eight-layer body.
(7) Next, the result was dried (vitrified) using a clean air dryer in an incubator at 10°C and humidity 40%.
(8) Next, UV irradiation (total UV irradiation amount per unit area: 400 mJ/cm²) was performed. Furthermore, after UV irradiation, the atelocollagen Vitrigel (registered trademark) membrane eight-layer adhesive dried body was inverted and UV irradiation (total UV irradiation amount per unit area: 400 mJ/cm²) was performed.
(9) An atelocollagen Vitrigel (registered trademark) membrane eight-layer adhesive dried body having a diameter of 13 mm was rehydrated with PBS. Next, using the punch with a diameter of 8 mm, the punch was placed in a concentric state with an atelocollagen Vitrigel (registered trademark) membrane eight-layer adherent having a diameter of 13 mm to cut out an atelocollagen Vitrigel (registered trademark) membrane eight-layer adherent with a diameter of 8 mm to produce a ring-shaped atelocollagen Vitrigel (registered trademark) membrane eight-layer adherent (inner diameter 8 mm, outer diameter 13 mm).
(10) Next, by carrying out drying (vitrifying) using a clean air dryer in an incubator at 10°C and with a humidity of 40%, a ring-shaped atelocollagen Vitrigel (registered trademark) membrane eight-layer adhesive dried body (inner diameter 8 mm, outer diameter 13 mm) was produced.
(11) Next, one sheet of an atelocollagen Vitrigel (registered trademark) membrane dried body was produced using the same method as in (1) of Production Example 7. Next, the one produced atelocollagen Vitrigel (registered trademark) membrane dried body was rehydrated with PBS to prepare an atelocollagen Vitrigel (registered trademark) membrane. Next, the atelocollagen Vitrigel (registered trademark) membrane was cut out using a punch having a diameter of 13 mm to produce an atelocollagen Vitrigel (registered trademark) membrane having a diameter of 13 mm.
   On the other hand, the ring-shaped atelocollagen Vitrigel (registered trademark) membrane eight layer adhered dried body (inner diameter 8 mm, outer diameter 13 mm) produced in (10) was also rehydrated with PBS to prepare ring-shaped atelocollagen Vitrigel (registered trademark) membrane eight-layer adherent (inner diameter 8 mm, outer diameter 13 mm).
(12) An atelocollagen Vitrigel (registered trademark) membrane with a diameter of 13 mm was stretched on a vinyl sheet, and 90 µL of 0.5% atelocollagen sol was added thereon to spread out so as to not protrude. Next, a ring-shaped atelocollagen Vitrigel (registered trademark) membrane eight-layer adherent (inner diameter 8 mm, outer diameter 13 mm) prepared in (11) was overlaid thereon.
(13) Next, the result was dried (vitrified) using a clean air dryer in an incubator at 10°C and 40% humidity.
(14) Next, UV irradiation (total UV irradiation amount per unit area: 400 mJ/cm²) was performed with the surface on which the 13 mm diameter atelocollagen Vitrigel (registered trademark) membrane dried body was pasted being on top.
(15) A ring-shaped atelocollagen Vitrigel (registered trademark) membrane eight layer adhered dried body (inner diameter 8 mm, outer diameter 13 mm) to which an atelocollagen Vitrigel (registered trademark) membrane dried body was adhered on one side was rehydrated with PBS. Then, an appropriate amount of 0.5% atelocollagen sol was added and spread so as to not protrude on the ring surface on the side on which the atelocollagen Vitrigel (registered trademark) membrane was not adhered.
(16) Next, one sheet of atelocollagen Vitrigel (registered trademark) membrane dried body was produced using the same method as in (1) of Production Example 7. Then, an atelocollagen Vitrigel (registered trademark) membrane was prepared by rehydrating one sheet of the produced atelocollagen Vitrigel (registered trademark) membrane dried body with PBS. Next, the atelocollagen Vitrigel (registered trademark) membrane was cut out using a punch having a diameter of 13 mm to produce an atelocollagen Vitrigel (registered trademark) membrane having a diameter of 13 mm. Next, an atelocollagen Vitrigel (registered trademark) membrane with a diameter of 13 mm was stretched on the vinyl sheet. A surface, which was coated with atelocollagen sol, of a ring-shaped atelocollagen Vitrigel (registered trademark) membrane eight-layer adherent (inner diameter 8 mm, outer diameter 13 mm) to which an atelocollagen Vitrigel (registered trademark) membrane dried body was adhered to one side prepared in (15) were covered on this atelocollagen Vitrigel (registered trademark) membrane so as to be adhered thereto.
(17) Next, the result was dried (vitrified) using a clean air dryer in an incubator at 10°C and 40% humidity.
(18) Next, UV irradiation (total UV irradiation amount per unit area: 400 mJ/cm²) was performed with the surface on which the 13 mm diameter atelocollagen Vitrigel (registered trademark) membrane dried body was newly pasted being on top.
(19) Next, vitrification was allowed to proceed in a dish or the like to produce a cell enclosure device (refer to FIG. 30A) to which an atelocollagen Vitrigel (registered trademark) membrane dried body having a diameter of 13 mm was adhered on both surfaces of a ring-shaped atelocollagen Vitrigel (registered trademark) membrane eight layer adhered dried body (inner diameter: 8 mm, outer diameter: 13 mm).
(20) Next, with respect to the obtained cell enclosure device, an indwelling needle was punctured in the ceiling surface of the cell enclosure device, the inner needle was removed, and the indwelling needle catheter was attached. Next, it was confirmed that, using this indwelling needle catheter, it is possible to safely inject a suspension of human dermal fibroblasts and to enclose cells (refer to FIG. 30B).

### [Example 17] Production of Dermal Tissue-Type Chip Using Human Dermis-Derived Fibroblasts 3

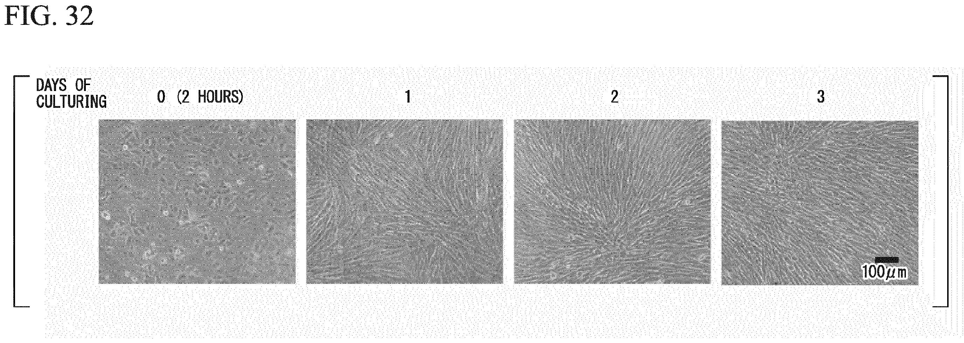

(1) Pre-cultured human dermal fibroblasts were collected and mixed with the culture medium so as to be 5.2 × 10⁵ cells/mL to prepare a suspension of human dermal fibroblasts.
(2) Next, an indwelling needle was punctured from an atelocollagen Vitrigel (registered trademark) membrane dried body on the ceiling side of the cell enclosure device produced in Production Example 8, which an atelocollagen Vitrigel (registered trademark) membrane dried body with a diameter of 13 mm was adhered to both surfaces of a ring-shaped atelocollagen Vitrigel (registered trademark) membrane eight layer adhered dried body (inner diameter: 8 mm, outer diameter: 13 mm), and subsequently the inner needle was removed, resulting that an indwelling needle catheter was attached. Next, a syringe was connected to the indwelling needle catheter, and 140 µL of a suspension (5.2 × 10⁵ cells/mL) of human dermal fibroblasts was injected to produce a dermal tissue-type chip. Next, the indwelling needle catheter was lightly pulled and removed from the cell enclosure device. At this time, since the suspension injected into the cell enclosure device hardly leaked out, the initial seeding density was 1.45 × 10⁵/cm².
(3) Next, 5.0 mL of the culture medium was poured into a 6 cm diameter dish, and the dermal tissue-type chip produced in (2) was immersed in the culture medium and culturing was started. Thereafter, the culture medium in each well was exchanged every other day and cultured for approximately three days. FIG. 31 shows an image photographing a dermal tissue-type chip on day 1 of culturing.
(4) Morphological changes of multicellular populations were analyzed by observation over time with a phase contrast microscope. FIG. 32 shows a phase contrast microscope image of the dermal tissue-type chip on days 0 (2 hours), 1, 2, and 3 of culturing.

From FIG. 32, it was confirmed that human dermal fibroblasts proliferated satisfactorily after adhering to an atelocollagen Vitrigel (registered trademark) membrane.

### Industrial Applicability

The cell enclosure device of the present embodiment is not only excellent in cell protection performance but is also easy to handle and makes the culturing of cells for long periods possible.

Furthermore, constructing a tissue-type chip, organ-type chip, or organ-type chip system using the cell enclosure device of the present embodiment makes it possible to expect effects such as drug efficacy against disease, confirmation tests for the metabolic pathways or cytotoxicity of drugs and their metabolites, and the like as a substitute for the culture models or animal experiments of the related art.

In addition, in a case where the cell enclosure device of the present embodiment is formed of a material having biocompatibility, it is possible to expect effects in regenerative medicine as a medical cell transplantation device.

In addition, it is possible to safely and reliably transport cells using the cell enclosure device of the present embodiment, and it is also possible to handle transportation for long periods.

In addition, it is possible for the cell enclosure device of the present embodiment to enclose, grow, or propagate cells other than animal cells or small living organisms.

### Reference Signs List

1: POROUS MEMBRANE
2: MEMBER
3: SUPPORT
4, 101: TUBE
10, 20, 30: CELL ENCLOSURE DEVICE
100, 200, 300, 400: TISSUE-TYPE CHIP
1A, 1B, 1C, ID: ORGAN-TYPE CHIP SYSTEM

## Claims

1. A cell enclosure device for constructing a multicellular structure obtained by culturing cells, comprising:
a porous membrane in at least a portion of the cell enclosure device.

2. The cell enclosure device according to claim 1,
wherein the porous membrane is a semipermeable membrane having liquid-tightness in a gas phase and a semipermeable property in a liquid phase.

3. The cell enclosure device according to claim 1 or 2,
wherein multicellular cells suspended in a culture medium are able to be injected and an internal volume is 10 mL or less.

4. The cell enclosure device according to claim 2 or 3,
wherein the entire device is formed of the semipermeable membrane.

5. The cell enclosure device according to any one of claims 2 to 4,
wherein the semipermeable membrane is formed of a material having biocompatibility.

6. The cell enclosure device according to claim 5,
wherein the material having biocompatibility is a component derived from an extracellular matrix available for gelation.

7. The cell enclosure device according to claim 6,
wherein the component derived from the extracellular matrix available for gelation is native collagen or atelocollagen.

8. A tissue-type chip comprising:
the cell enclosure device according to any one of claims 1 to 7, in which one type of cells is enclosed.

9. The tissue-type chip according to claim 8,
wherein a density of the cells is 2.0 × 10³ cells/mL or more and 1.0 × 10⁹ cells/mL or less.

10. An organ-type chip comprising:
the cell enclosure device according to any one of claims 1 to 7, in which at least two types of cells are enclosed.

11. The organ-type chip according to claim 10,
wherein a density of the cells is 2.0 × 10³ cells/mL or more and 1.0 × 10⁹ cells/mL or less.

12. A kit for providing a multicellular structure, comprising:
an openable and closable sealed container including the tissue-type chip according to claim 8 or 9 or the organ-type chip according to claim 10 or 11, and a culture medium.

13. An organ-type chip system comprising:
at least two of
the tissue-type chip according to claim 8 or 9, or
the organ-type chip according to claim 10 or 11,
wherein the tissue-type chips or the organ-type chips are connected while maintaining a cell enclosure property.

14. A method for culturing cells, the method comprising:
using the cell enclosure device according to any one of claims 1 to 7.

15. A cell transportation method comprising:
using cells in the cell enclosure device according to any one of claims 1 to 7.
